# EUROPEAN PATENT APPLICATION

(11) **EP 3 339 297 A1**
(43) Date of publication of application: **27.06.2018**
(21) Application number: 16205470.4
(22) Date of filing: 20.12.2016
(51) Int. Cl.: C07D 271/06, C07D 413/02, C07D 413/14, C07D 413/10, C07D 413/12, A01N 43/82

(54) **SUBSTITUTED OXADIAZOLES FOR COMBATING PHYTOPATHOGENIC FUNGI**

(71) Applicant: BASF SE, 67056 Ludwigshafen am Rhein (DE)
(72) Inventor: Grammenos, Wassilios, 67056 Ludwigshafen (DE); Terteryan-Seiser, Violeta, 67056 Ludwigshafen (DE); Quintero Palomar, Maria Angelica, 67117 Limburgerhof (DE); Craig, Ian Robert, 67056 Ludwigshafen (DE); Wiebe, Christine, 67056 Ludwigshafen (DE); Fehr, Marcus, 67117 Limburgerhof (DE); Mentzel, Tobias, 67117 Limburgerhof (DE); Escribano Cuesta, Ana, 67056 Ludwigshafen (DE); Winter, Christian Harald, 400705 Navi Mumbai (IN); Lohmann, Jan Klaas, 67056 Ludwigshafen (DE); Müller, Bernd, 67056 Ludwigshafen (DE); Cambeis, Erica, 67056 Ludwigshafen (DE); Grote, Thomas, 67056 Ludwigshafen (DE)
(74) Representative: BASF IP Association

(57) **Abstract**

The present invention relates to novel oxadiazoles of the formula I, or an N-oxide and/or their agriculturally useful salts and to their use for controlling phytopathogenic fungi, or to a method for combating phytopathogenic harmful fungi, which process comprises treating the fungi or the materials, plants, the soil or seeds to be protected against fungal attack, with an effective amount of at least one compound of the formula I, or an N-oxide, or an agriculturally acceptable salt thereof; the present invention also relates to mixtures comprising at least one such compound of the formula I and at least one further pesticidally active substance selected from the group consisting of herbicides, safeners, fungicides, insecticides, and plant growth regulators; the present invention also relates to agrochemical compositions comprising at least one such compound of the formula I and to agrochemical compositions further comprising seeds.

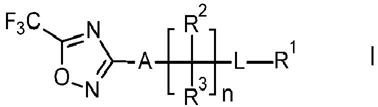

## Description

The present invention relates to novel oxadiazoles of the formula I, or an N-oxide and/or their agriculturally useful salts and to their use for controlling phytopathogenic fungi, or to a method for combating phytopathogenic harmful fungi, which process comprises treating the fungi, the plants, the soil or seeds to be protected against fungal attack, with an effective amount of at least one compound of the formula I, or an N-oxide, or an agriculturally acceptable salt thereof; the present invention also relates to mixtures comprising at least one such compound of the formula I and at least one further pesticidally active substance selected from the group consisting of herbicides, safeners, fungicides, insecticides, and plant growth regulators; the present invention also relates to agrochemical compositions comprising at least one such compound of the formula I and to agrochemical compositions further comprising seeds.

EP 276432 A2 relates to 3-phenyl-5-trifluoromethyloxadiazole derivatives and to their use to combat phytopathogenic microorganisms. WO 2015/185485 describes the use of certain substituted oxadiazoles for combating phytopathogenic fungi.

In many cases, in particular at low application rates, the fungicidal activity of known fungicidal compounds is unsatisfactory. Based on this, it was an objective of the present invention to provide compounds having improved activity and/or a broader activity spectrum against phytopathogenic fungi. This objective is achieved by the oxadiazoles of the formula I and/or their agriculturally useful salts for controlling phytopathogenic fungi.

The compounds according to the invention differ from those described in state of the art documents mentioned above in the nature of the -CR²R³-L-R¹ moiety.

Accordingly, the present invention relates to the compounds of the formula I, or the N-oxides, or the agriculturally acceptable salts thereof, 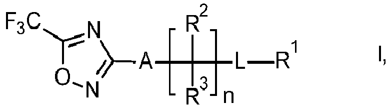 wherein:
- A: is phenyl; and wherein the phenyl ring is unsubstituted or substituted with 1, 2, 3 or 4 identical or different groups R^{A}; wherein
R^{A} is halogen, cyano, C₁-C₆-alkyl, Cₗ-C₆-haloalkyl, C₁-C₆-alkoxy or C₁-C₆-haloalkoxy;
- L: is -S(=O)ₚ- or -S(=N-R^{L})(=O);
- p: is 0,1 or 2;
- R^{L}: is hydrogen, cyano or C₁-C₃-alkyl;
- R¹: is C₁-C₆-alkyl, C₁-C₆-alkoxy, C₂-C₆-alkenyl, C₂-C₆-alkynyl, C₃-C₈-cycloalkyl, C₃-C₈-cycloalkoxy, C₃-C₈-cycloalkenyl, phenyl-C₁-C₄-alkyl, heteroaryl-C₁-C₄-alkyl, phenyl, naphthyl or a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms; and wherein 1 or 2 carbon ring member atoms of the heterocycle may be replaced by 1 or 2 groups independently selected from -C(=O)- and -C(=S)-; and wherein the heteroaryl group in heteroaryl-C₁-C₄-alkyl is a 5- or 6-membered aromatic heterocycle, wherein the ring member atoms of the heterocyclic ring include besides carbon atoms 1, 2, 3 or 4 heteroatoms selected from N, O and S as ring member atoms; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl, Cₗ-C₆-haloalkyl, C₁-C₆-alkoxy, C₁-C₆-haloalkoxy and C₃-C₈-cycloalkyl;
- R², R³: each single radical individually and independently of each other, are selected from the group consisting of hydrogen, halogen, cyano, C₁-C₄-alkyl, C₁-C₄-alkenyl, C₁-C₄-alkynyl, C₁-C₄-haloalkyl, C₁-C₄-alkoxy and C₁-C₄-haloalkoxy;
- or R² and R³: together with the carbon atom to which they are bound form a saturated 3- to 7-membered carbocycle or a saturated 3- to 6-membered heterocycle; wherein the saturated heterocycle includes beside carbon atoms 1, 2 or 3 heteroatoms independently selected from the group consisiting of N, O and S as ring member atoms; and wherein said N ring member atom is substituted with the group R^{N}; and wherein
R^{N} is hydrogen, C₁-C₆-alkyl or halogen;
and wherein said S ring member atom is unsubstituted or substituted with 1 or 2 oxo radicals; and wherein one or two CH₂ groups of the saturated carbocycle or of the saturated heterocycle may be replaced by one or two groups independently selected from -C(=O)- and -C(=S)-; and wherein the carbon ring member atoms of the saturated carbocycle or the saturated heterocycle are unsubstituted or substituted with a total number of 1, 2, 3, 4 or up to the maximum possible number of identical or different radicals selected from the group consisting of halogen, cyano, C₁-C₆-alkyl, C₁-C₆-haloalkyl, C₁-C₆-alkoxy, C₁-C₆-haloalkoxy and C₃-C₈-cycloalkyl; and
- n: is 0, 1 or 2.

Agriculturally acceptable salts of the compounds of the formula I encompass especially the salts of those cations or the acid addition salts of those acids whose cations and anions, respectively, have no adverse effect on the fungicidal action of the compounds I. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may be substituted with one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of acceptable acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting a compound I with an acid of the corresponding anion, preferably of hydrochloric acid, hydrobromic acid, sulfuric acid, phosphoric acid or nitric acid.

Compounds of the formula I can exist as one or more stereoisomers. The various stereoisomers include enantiomers, diastereomers, atropisomers arising from restricted rotation about a single bond of asymmetric groups and geometric isomers. They also form part of the subject matter of the present invention. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. The compounds of the invention may be present as a mixture of stereoisomers, e.g. a racemate, individual stereoisomers, or as an optically active form.

Compounds of the formula I can be present in different crystal modifications whose biological activity may differ. They also form part of the subject matter of the present invention.

In respect of the variables, the embodiments of the intermediates obtained during preparation of compounds I correspond to the embodiments of the compounds of formula I. The term "compounds I" refers to compounds of formula I.

In the definitions of the variables given above, collective terms are used which are generally representative for the substituents in question. The term "Cₙ-Cₘ" indicates the number of carbon atoms possible in each case in the substituent or substituent moiety in question.

The moieties having two ore more possibilities to be attached apply following:
The moieties having no brackets in the name are bonded via the last moiety e.g. C₂-C₆-alkenyloxyimino-C₁-C₄-alkyl is bonded via C₁-C₄-alkyl, heteroaryl-C₁-C₄-alkyl is bonded via C₁-C₄-alkyl etc.

The moieties having brackets in the name are bonded via the first moiety e.g. C(=O)-(C₁-C₆-alkyl) is bonded via C=O, etc.

The term "halogen" refers to fluorine, chlorine, bromine and iodine.

The term "C₁-C₆-alkyl" refers to a straight-chained or branched saturated hydrocarbon group having 1 to 6 carbon atoms, for example methyl, ethyl, propyl, 1-methylethyl, butyl, 1-methylpropyl, 2-methylpropyl, and 1,1-dimethylethyl.

The term "C₁-C₆-haloalkyl" refers to a straight-chained or branched alkyl group having 1 to 6 carbon atoms (as defined above), wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "C₁-C₆-alkoxy" refers to a straight-chain or branched alkyl group having 1 to 6 carbon atoms (as defined above) which is bonded via an oxygen, at any position in the alkyl group, for example methoxy, ethoxy, n-propoxy, 1-methylethoxy, butoxy, 1-methylpropoxy, 2-methylpropoxy or 1,1-dimethylethoxy.

The term "C₁-C₆-haloalkoxy" refers to a C₁-C₆-alkoxy group as defined above, wherein some or all of the hydrogen atoms may be replaced by halogen atoms as mentioned above, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromo¬ethoxy, 4-fluorobutoxy, 4-chlorobutoxy, 4-bromobutoxy or nonafluorobutoxy.

The terms "phenyl-C₁-C₄-alkyl or heteroaryl-C₁-C₄-alkyl" refer to alkyl having 1 to 4 carbon atoms (as defined above), wherein one hydrogen atom of the alkyl radical is replaced by a phenyl or hetereoaryl radical respectively.

The term "C₂-C₆-alkenyl" refers to a straight-chain or branched unsaturated hydrocarbon radical having 2 to 6 carbon atoms and a double bond in any position, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond, such as ethynyl, 1-propynyl, 2-propynyl (propargyl), 1-butynyl, 2-butynyl, 3-butynyl, 1-methyl-2-propynyl.

The term "C₃-C₈-cycloalkyl" refers to monocyclic saturated hydrocarbon radicals having 3 to 8 carbon ring members such as cyclopropyl (C₃H₅), cyclobutyl, cyclopentyl, cyclohexyl, cycloheptyl or cyclooctyl.

The term "C₃-C₈-cycloalkyloxy" refers to a cycloalkyl radical having 3 to 8 carbon atoms (as defined above), which is bonded via an oxygen.

The term "aliphatic" refers to compounds or radicals composed of carbon and hydrogen and which are non-aromatic compounds. An alicyclic compound or radical is an organic compound that is both aliphatic and cyclic. They contain one or more all-carbon rings which may be either saturated or unsaturated, but do not have aromatic character.

The term "aryl" refers to aromatic monocyclic or polycyclic ring systems incuding besides carbon atoms no heteroatom or 1, 2, 3 or 4 heteroatoms independently selected from the group consisting of N, O and S.

The term "heteroaryl" refers to aromatic monocyclic or polycyclic ring systems incuding besides carbon atoms, 1, 2, 3 or 4 heteroatoms independently selected from the group consisting of N, O and S.

The terms "cyclic moiety" or "cyclic group"refer to a radical which is an alicyclic ring or an aromatic ring, such as, for example, phenyl or heteroaryl.

The term "and wherein any of the aliphatic or cyclic groups are unsubstituted or substituted with 1, 2, 3, 4 or up to the maximum possible number of identical or different radiclas" refers to aliphatic groups, cyclic groups and groups, which contain an aliphatic and a cyclic moiety in one group, such as in, for example, phenyl-C₁-C₄-alkyl; therefore a group which contains an aliphatic and a cyclic moiety both of these moieties may be substituted or unsubstituted independently of each other.

The term "phenyl" refers to an aromatic ring system incuding six carbon atoms (commonly referred to as benzene ring). In association with the group A the term "phenyl" is to be interpreted as a benzene ring or phenylene ring, which is attached to both, the oxadiazole moiety and the -CR²R³-L-R¹ group.

The term "R², R³ each single radical individually and independently of each other, are selected from the group consisting of..." means that each radical R² or R³, irrespective of whether there is one or more of such radical present (n = 1 or 2), are independently selected from the group of substituents as defined herein; as a result two radicals R² (n = 2), for example, may hence be identical or different.

The term "saturated 3- to 7-membered carbocycle" is to be understood as meaning saturated carbocycles having 3, 4, 5, 6 or 7 ring member carbon atoms. Examples include cyclopropyl, cyclopentyl, cyclohexyl, cycloheptyl, cycloheptenyl and the like.

The term "3- to 6-membered saturated heterocycle, wherein the saturated heterocycle includes beside carbon atoms 1, 2 or 3 heteroatoms selected from the group consisting of N, O and S as ring member atoms", is to be understood as meaning, for example:
a 3- or 4-membered saturated heterocycle which contains 1 or 2 heteroatoms from the group consisting of N, O and S as ring members such as oxirane, aziridine, thiirane, oxetane, azetidine, thiethane, [1,2]dioxetane, [1,2]dithietane, [1,2]diazetidine;
and a 5- or 6-membered saturated heterocycle such as 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetrahydro[2H]azepin-2-,-3-,-4-,-5-,-6-or-7-yl, 2,3,4,7-tetrahydro[1H]azepin-1-,-2-,-3-,-4-,-5-,-6- or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-,-6- or-7-yl, hexahydroazepin-1-,-2-,-3-or-4-yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding -ylidene radicals.

The term "5- or 6-membered heteroaryl" or the term "5- or 6-membered aromatic heterocycle" refer to aromatic ring systems incuding besides carbon atoms, 1, 2, 3 or 4 heteroatoms independently selected from the group consisting of N, O and S, for example, a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl;
or a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

In respect of the variables, the embodiments of the intermediates correspond to the embodiments of the compounds I. Preference is given to those compounds I and, where applicable, also to compounds of all sub-formulae provided herein, wherein all variables have independently of each other or more preferably in combination (any possible combination of 2 or more substituents as defined herein) the following meanings:
In one embodiment of the invention A is phenyl, which is unsubstituted or substituted with 1, 2, 3 or 4 identical or different groups R^{A} as defined or preferably defined herein and wherein the - CR²R³-L-R¹ group is attached to the phenyl ring in *para*-position with regard to the trifluoromethyloxadiazole group.

In one aspect of the invention A is phenyl, which is unsubstituted or substituted with 1, 2, 3 or 4 identical or different groups R^{A} as defined or preferably defined herein and wherein the -CR²R³-L-R¹ group is attached to the phenyl ring in *meta*-position with regard to the trifluoromethyloxadiazole group.
In a further aspect of the invention A is phenyl, which is substituted with 1 or 2 identical or different groups R^{A} as defined or preferably defined herein and wherein the -CR²R³-L-R¹ group is attached to the phenyl ring in *para*-position with regard to the trifluoromethyloxadiazole group. In another aspect of the invention A is phenyl, which is unsubstituted and wherein the -CR²R³-L-R¹ group is attached to the phenyl ring in *para*-position with regard to the trifluoromethyloxadiazole group.

In a preferred embodiment of the invention R^{A} is halogen, cyano or C₁-C₆-alkyl; in particular halogen or C₁-C₆-alkyl. In a more preferable embodiment R^{A} is chlorine, fluorine or methyl.
In a further preferred embodiment of the invention A is substituted with halogen, in particular one halogen. In a special preferred embodiment of the invention A is substituted with fluorine, in particular one fluorine. In a further special preferred embodiment of the invention A is substituted with chlorine, in particular one chlorine.
In a further preferred embodiment of the invention A is substituted with C₁-C₆-alkyl. In a special preferred embodiment of the invention A is substituted with a methyl group, in particular one methyl group. In a further special preferred embodiment of the invention A is substituted with an ethyl group, in particular one ethyl group. In a further special preferred embodiment of the invention A is unsubstituted.

L according to one embodiemnt is -S(=O)ₚ- or -S(=N-R^{L})(=O)-; wherein p is 1 or 2. In one embodiment p is 0. In another embodiment p is 1. In a preferred embodiment p is 2. In another aspect L is -S(=N-CN)(=O)- or -S(=N-CH₃)(=O)-.

According to one embodiment R¹ is C₁-C₆-alkyl, preferably methyl, ethyl, n-propyl, i-propyl, n-butyl, 1-methyl-propyl, 2-methyl-propyl, t-butyl, in particular methyl or ethyl.
According to a further embodiment R¹ is C₂-C₆-alkenyl, preferably ethylenyl or allyl.
According to a further embodiment R¹ is C₂-C₆-alkynyl, preferably ethynyl or 1-propynyl. According to a further embodiment R¹ is C₃-C₈-cycloalkyl, preferrably cyclopropyl, cyclobutyl, cyclopentyl or cyclohexyl; in particular cyclopropyl.
According to one embodiment R¹ is C₁-C₆-alkyl substituted with halogen, wherein alkyl is preferably methyl, ethyl, n-propyl, i-propyl, n-butyl, 1-methyl-propyl, 2-methyl-propyl, t-butyl, in particular methyl or ethyl.
According to one embodiment R¹ is C₁-C₆-alkyl substituted with fluorine, wherein alkyl is preferably methyl, ethyl, n-propyl, i-propyl, n-butyl, 1-methyl-propyl, 2-methyl-propyl, t-butyl, in particular methyl or ethyl. In a preferred embodiemnt R¹ is CH₂CF₃, CH₂CHF₂, CF₂CF₃, CHCH₃CF₃, CH₂CF₂CH₃, CH₂CF(CH₃)₂, CH₂CH₂CF₃, CH₂CHCH₃CF₃, CH₂C(CH₃)₂CF₃ or CF₂CF₂CF₃. In a special embodiment of the invention, R¹ is CF₃.
According to one embodiment R¹ is phenyl-C₁-C₄-alkyl, wherein alkyl is preferably methyl or ethyl, in particular methyl.
According to one embodiment R¹ is heteroaryl-C₁-C₄-alkyl,, wherein alkyl is preferably methyl or ethyl, in particular methyl; and wherein heteroaryl is pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl, 1,2,4-thiadiazol-3-yl, 1,2,4-thiadiazol-5-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl or 1,2,4-triazin-3-yl.
According to one embodiment R¹ is phenyl. According to a further embodiment R¹ is benzyl. According to a further embodiment R¹ is naphthyl. According to one embodiment R¹ is a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms and wherein 1 or 2 carbon ring member atoms of the heterocycle may be replaced by 1 or 2 groups independently selected from C(=O) and C(=S).
According to a further embodiment the above mentioned groups for R¹ may be substituted with 1, 2, 3 or 4 identical or different substituents selected from the group consisting of methyl, ethyl, propyl, butyl, preferably methyl. According to a further embodiment the above mentioned groups for R¹ may be substituted with 1, 2, 3 or 4 identical or different substituents selected from the group consisting of partially or fully halogenated methyl, ethyl, propyl, butyl, preferably partially or fully halogenated methyl, in particular CF₃.

According to a further embodiment the above mentioned groups for R¹ may be substituted with 1, 2, 3 or 4 identical or different substituents selected from the group consisting of methoxy, ethoxy, propoxy, butoxy, preferably methoxy. According to a further embodiment the above mentioned groups for R¹ may be substituted with 1, 2, 3 or 4 identical or different substituents selected from the group consisting of partially or fully halogenated methoxy, ethoxy, propoxy, butoxy, preferably partially or fully halogenated methoxy, in particular OCF₃.

According to yet another embodiment the cyclic moieties in the above mentioned groups for R¹ may be unsubstituted or substituted with 1, 2, 3 or 4 identical or different halogen atoms, in particular with fluorine atoms, more particularly with 1 or 2 fluorine atoms.

In a special embodiment of the invention, R¹ is phenyl, which is unsubstituted or substituted with 1, 2, 3 or 4 identical or different radicals selected from the group consisting of chlorine, fluorine, bromine, iodine, methyl, methoxy, CF₃ or OCF₃, in particular selected from halogen atoms, more particularly substituted with 1 or 2 fluorine atoms.

In a further special embodiment of the invention R¹ is benzyl (-CH₂-phenyl), wherein the phenyl ring is unsubstituted or substituted with 1, 2, 3 or 4 identical or different radicals selected from the group consisting of chlorine, fluorine, bromine, iodine, methyl, methoxy, CF₃ or OCF₃, in particular selected from halogen atoms, more particularly substituted with 1 or 2 fluorine atoms. In a further special embodiment R¹ is naphtyl, which is unsubstituted or substituted with 1, 2, 3 or 4 identical or different radicals selected from the group consisting of chlorine, fluorine, bromine, iodine, methyl, methoxy, CF₃ or OCF₃, in particular selected from halogen atoms, more particularly substituted with 1 or 2 fluorine atoms.

In a further special embodiment of the invention R¹ is a heteroaryl group selected from thiophene, pyrrole, pyrazole, oxadiazole, pyridine and pyrimidine; and wherein the heteroaryl group is unsubstituted or substituted with 1, 2, 3 or 4 identical or different radicals selected from the group consisting of chlorine, fluorine, bromine, iodine, methyl, methoxy, CF₃ or OCF₃, in particular selected from halogen atoms, more particularly substituted with 1 or 2 fluorine atoms. In one embeodiment of the invention R¹ is C₁-C₆-alkyl, C₁-C₆-alkoxy, C₂-C₆-alkenyl, C₂-C₆-alkynyl, C₃-C₈-cycloalkyl, C₃-C₈-cycloalkoxy, C₃-C₈-cycloalkenyl, phenyl-C₁-C₄-alkyl, heteroaryl-C₁-C₄-alkyl or phenyl; and wherein the heteroaryl group in heteroaryl-C₁-C₄-alkyl is a 5- or 6-membered aromatic heterocycle, wherein the ring member atoms of the heterocyclic ring include besides carbon atoms 1, 2, 3 or 4 heteroatoms selected from N, O and S as ring member atoms; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl, C₁-C₆-haloalkyl, C₁-C₆-alkoxy, C₁-C₆-haloalkoxy and C₃-C₈-cycloalkyl.

In another aspect R¹ is C₁-C₆-alkyl, C₃-C₈-cycloalkyl, phenyl-C₁-C₄-alkyl or phenyl; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl, C₁-C₆-haloalkyl, C₁-C₆-alkoxy, C₁-C₆-haloalkoxy and C₃-C₈-cycloalkyl.

In one embodiment R¹ is C₁-C₆-alkyl, Cₗ-C₆-haloalkyl, C₃-C₈-cycloalkyl or phenyl; wherein the cyclic groups are unsubstituted or substituted with 1, 2, 3 or 4 or up to the maximum possible number of identical or different radicals selected from the group consisiting of chlorine, fluorine, methyl, methoxy, CF₃ or OCF₃; in particular selected from halogen atoms.

In a preferred embodiment R¹ is methyl, ethyl, n-propyl, i-propyl, n-butyl, 1-methyl-propyl, 2-methyl-propyl, t-butyl, cyclopropyl, cyclobutyl, cyclopentyl or phenyl; wherein the aliphatic and cyclic groups are unsubstituted or substituted with 1 or 2 identical or different radicals selected from the group consisiting of chlorine and fluorine.

In a special embodiment R¹ is C₁-C₆-alkyl, C₁-C₆-haloalkyl or C₃-C₈-cycloalkyl.

In a special embodiment R¹ is methyl, ethyl, n-propyl, i-propyl, n-butyl, 1-methyl-propyl, 2-methyl-propyl, cyclopropyl, cyclobutyl or cyclopentyl.

In one embodiment the invention relates to compounds of the formula I, wherein R² and R³ independently of each other are hydrogen, halogen, C₁-C₆-alkyl or C₁-C₆-haloalkyl.

In a further embodiment R² and R³ are independently of each other hydrogen, fluorine, methyl or trifluoromethyl.

In another aspect R² and R³ are both hydrogen.

In a further aspect R² is hydrogen and R³ is methyl.

In yet another aspect R² and R³ are both methyl.

In a further aspect R² and R³ are both fluorine.

In another aspect n is 1, R² and R³ are both hydrogen and R¹ is methyl, ethyl, n-propyl, i-propyl, n-butyl, 1-methyl-propyl, 2-methyl-propyl, cyclopropyl, cyclobutyl or cyclopentyl.

In a further aspect n is 1, R² is hydrogen, R³ is methyl and R¹ is methyl, ethyl, n-propyl, i-propyl, n-butyl, 1-methyl-propyl, 2-methyl-propyl, cyclopropyl, cyclobutyl or cyclopentyl.

In yet another aspect n is 1, R² and R³ are both methyl hydrogen and R¹ is methyl, ethyl, n-propyl, i-propyl, n-butyl, 1-methyl-propyl, 2-methyl-propyl, cyclopropyl, cyclobutyl or cyclopentyl. In a further aspect n is 1, R² and R³ are both fluorine and R¹ is methyl, ethyl, n-propyl, i-propyl, n-butyl, 1-methyl-propyl, 2-methyl-propyl, cyclopropyl, cyclobutyl or cyclopentyl.

In one embodiment R² and R³ together with the carbon atom to which they are bound form a vinyl group or a saturated monocyclic 3- to 5-membered saturated heterocycle or 3- to 5-membered saturated carbocycle; and wherein the saturated heterocycle includes beside one or more carbon atoms no heteroatoms or 1 or 2 heteroatoms independently selected from N, O and S as ring member atoms; and wherein the vinyl group, the heterocycle or the carbocycle is unsubstituted or substituted with 1, 2, 3, 4 or up to the maximum possible number of identical or different radicals selected from the group consisting of halogen, cyano, C₁-C₆-alkyl, C₁-C₆-haloalkyl, C₁-C₆-alkoxy, C₁-C₆-haloalkoxy and C₃-C₈-cycloalkyl; in particular selected from halogen, cyano and C₁-C₂-alkyl.

In one embodiment the invention relates to the compounds of the formula I, or the N-oxides, or the agriculturally acceptable salts thereof, wherein R² and R³ together with the carbon atom to which they are bound form a cyclopropyl, cyclobutyl or oxirane ring; and wherein the carbon ring member atoms of the cyclic groups are unsubstituted or substituted with 1, 2, 3, 4 or up to the maximum possible number of identical or different radicals selected from the group consisting of halogen, cyano, C₁-C₆-alkyl, Cₗ-C₆-haloalkyl, C₁-C₆-alkoxy, C₁-C₆-haloalkoxy and C₃-C₈-cycloalkyl; in particular selected from halogen, cyano and C₁-C₂-alkyl.

In another embodiment R² and R³ together with the carbon atom to which they are bound form a vinyl group or a 3- or 4-membered carbocylic ring; and wherein the vinyl group or the carbocylic ring is unsubstituted or substituted with 1, 2, 3, 4 or up to the maximum possible number of identical or different radicals selected from the group consisting of halogen, cyano and C₁-C₂-alkyl.

In a further aspect R² and R³ together with the carbon atom to which they are bound form a vinyl group or a cyclopropyl group, wherein the vinyl group or the cyclopropyl group is unsubstituted. In a further aspect R² and R³ together with the carbon atom to which they are bound form a cyclopropyl group, wherein the cyclopropyl group is unsubstituted.

In still another embodiment R² and R³ together with the carbon atom to which they are bound form a saturated 3-membered heterocycle; wherein the heterocycle includes beside two carbon atoms one heteroatom selected from N-R^{N},S and O as ring member atoms; wherein R^{N} is hydrogen or C₁-C₆-alkyl; particularly preferred the heterocycle is unsubstituted.

In yet another embodiment R² and R³ together with the carbon atom to which they are bound form an oxirane ring.

In one aspect of the invention n is 0, 1 or 2. In another aspect n is 0 or 1. In a preferred aspect n is 0. In yet another preferred aspect n is 2. In a particularly preferred aspect n is 1.

In one embodiment the invention relates to compounds of the formulae I.1 or I.2, or the N-oxides, or the agriculturally acceptable salts thereof, wherein A and the radicals R¹, R² and R³ are as defined or preferably defined herein.

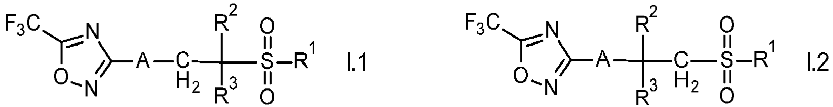

In a further aspect the present invention relates to compounds of the formulae I.A, I.B, I.C, I.D, I.E, I.F, I.G, I.H, I.J, I.K, I.L, I.M, I.N, I.O, I.P, I.Q, I.R, I.S, I.T, I.U, I.V or I.W, or the N-oxides, or the agriculturally acceptable salts thereof, or to their use for controlling phytopathogenic fungi; and wherein R¹ is as defined or preferably defined herein.

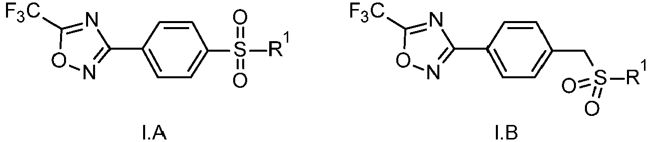

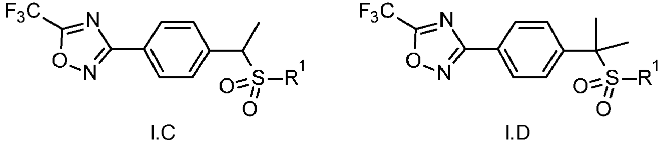

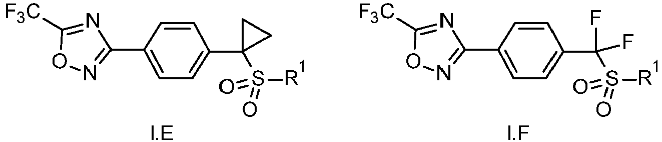

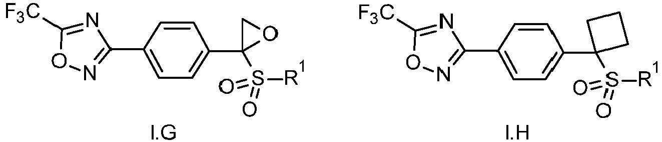

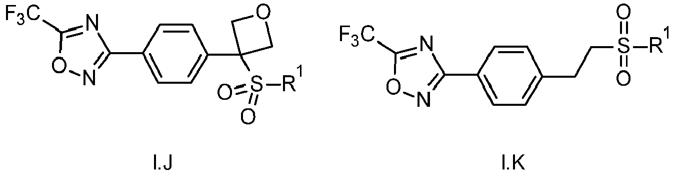

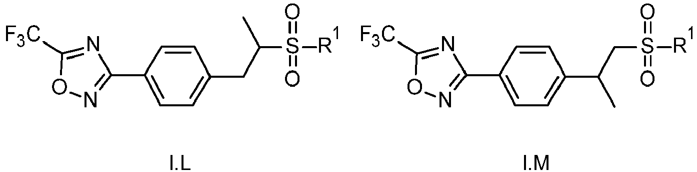

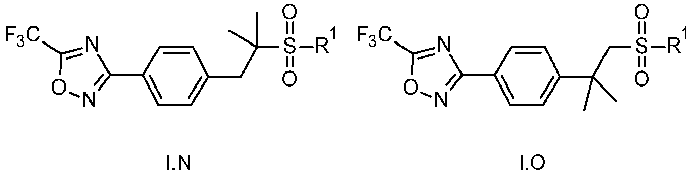

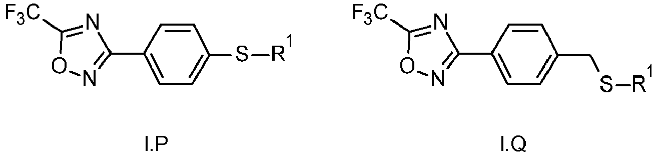

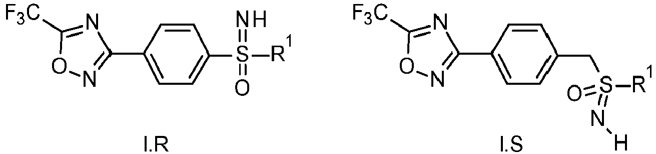

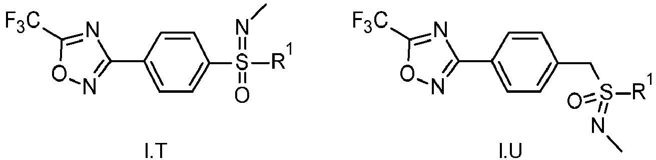

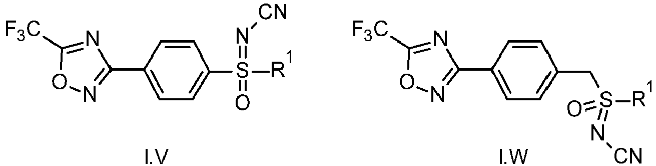

Preference is given to the compounds of the formulae I.A, I.B, I.C, I.D, I.E, I.F, I.G, I.H, I.J, I.K, I.L, I.M, I.N, I.O, I.P, I.Q, I.R, I.S, I.T, I.U, I.V or I.W, or the N-oxides, or the agriculturally acceptable salts thereof, compiled in Tables 1 to 22 below, and to their use according to the invention. The groups mentioned for a substituent in the tables are furthermore *per se,* independently of the combination in which they are mentioned, a particularly preferred aspect of the substituent in question.
Table 1 : Compounds of the formula I.A, in which R¹ for each individual compound corresponds in each case to one line of Table A (compounds I.A.A-1 to I.A.A-242). For example, compound I.A.A-3 is therefore to be construed as a compound of the formula I.A, wherein the meaning of R1 is as defined in line A-3 of table A (R¹ is CH₂CH₂CH₃, or n-propyl).
Table 2 : Compounds of the formula I.B, in which R¹ for each individual compound corresponds in each case to one line of Table A (compounds I.B.A-1 to I.B.A-242).
Table 3 : Compounds of the formula I.C, in which R¹ for each individual compound corresponds in each case to one line of Table A (compounds I.C.A-1 to I.C.A-242).
Table 4 : Compounds of the formula I.D, in which R¹ for each individual compound corresponds in each case to one line of Table A compounds (I.D.A-1 to I.D.A-242).
Table 5 : Compounds of the formula I.E, in which R¹ for each individual compound corresponds in each case to one line of Table A (compounds I.E.A-1 to I.E.A-242).
Table 6 : Compounds of the formula I.F, in which R¹ for each individual compound corresponds in each case to one line of Table A (compounds I.F.A-1 to I.F.A-242).
Table 7 : Compounds of the formula I.G, in which R¹ for each individual compound corresponds in each case to one line of Table A (compounds I.G.A-1 to I.G.A-242).
Table 8 : Compounds of the formula I.H, in which R¹ for each individual compound corresponds in each case to one line of Table A (compounds I.H.A-1 to I.H.A-242).
Table 9 : Compounds of the formula I.J, in which R¹ for each individual compound corresponds in each case to one line of Table A (compounds I.J.A-1 to I.J.A-242).
Table 10 : Compounds of the formula I.K, in which R¹ for each individual compound corresponds in each case to one line of Table A (compounds I.K.A-1 to I.K.A-242).
Table 11 : Compounds of the formula I.L, in which R¹ for each individual compound corresponds in each case to one line of Table A (compounds I.L.A-1 to I.L.A-242).
Table 12 : Compounds of the formula I.M, in which R¹ for each individual compound corresponds in each case to one line of Table A (compounds I.M.A-1 to I.M.A-242).
Table 13 : Compounds of the formula I.N, in which R¹ for each individual compound corresponds in each case to one line of Table A (compounds I.N.A-1 to I.N.A-242).
Table 14 : Compounds of the formula I.O, in which R¹ for each individual compound corresponds in each case to one line of Table A (I.O.A-1 to I.O.A-242).
Table 15 : Compounds of the formula I.P, in which R¹ for each individual compound corresponds in each case to one line of Table A (I.P.A-1 to I.P.A-242).
Table 16 : Compounds of the formula I.Q, in which R¹ for each individual compound corresponds in each case to one line of Table A (I.Q.A-1 to I.Q.A-242).
Table 17 : Compounds of the formula I.R, in which R¹ for each individual compound corresponds in each case to one line of Table A (I.R.A-1 to I.R.A-242).
Table 18 : Compounds of the formula I.S, in which R¹ for each individual compound corresponds in each case to one line of Table A (I.S.A-1 to I.S.A-242).
Table 19 : Compounds of the formula I.T, in which R¹ for each individual compound corresponds in each case to one line of Table A (I.T.A-1 to I.T.A-242).
Table 20 : Compounds of the formula I.U, in which R¹ for each individual compound corresponds in each case to one line of Table A (I.U.A-1 to I.U.A-242).
Table 21 : Compounds of the formula I.V, in which R¹ for each individual compound corresponds in each case to one line of Table A (I.V.A-1 to I.V.A-242).
Table 22 : Compounds of the formula I.W, in which R¹ for each individual compound corresponds in each case to one line of Table A (I.W.A-1 to I.W.A-242).

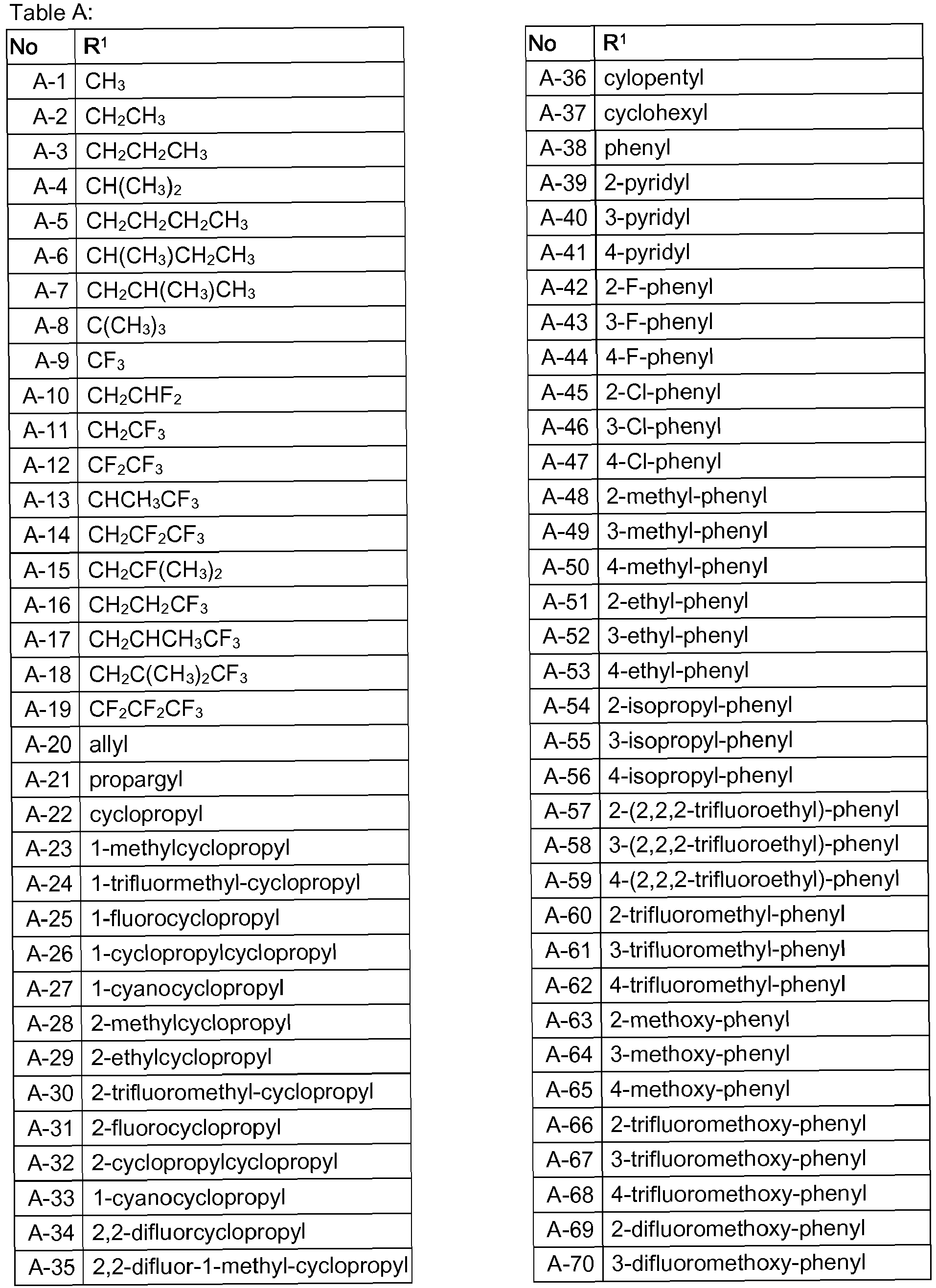

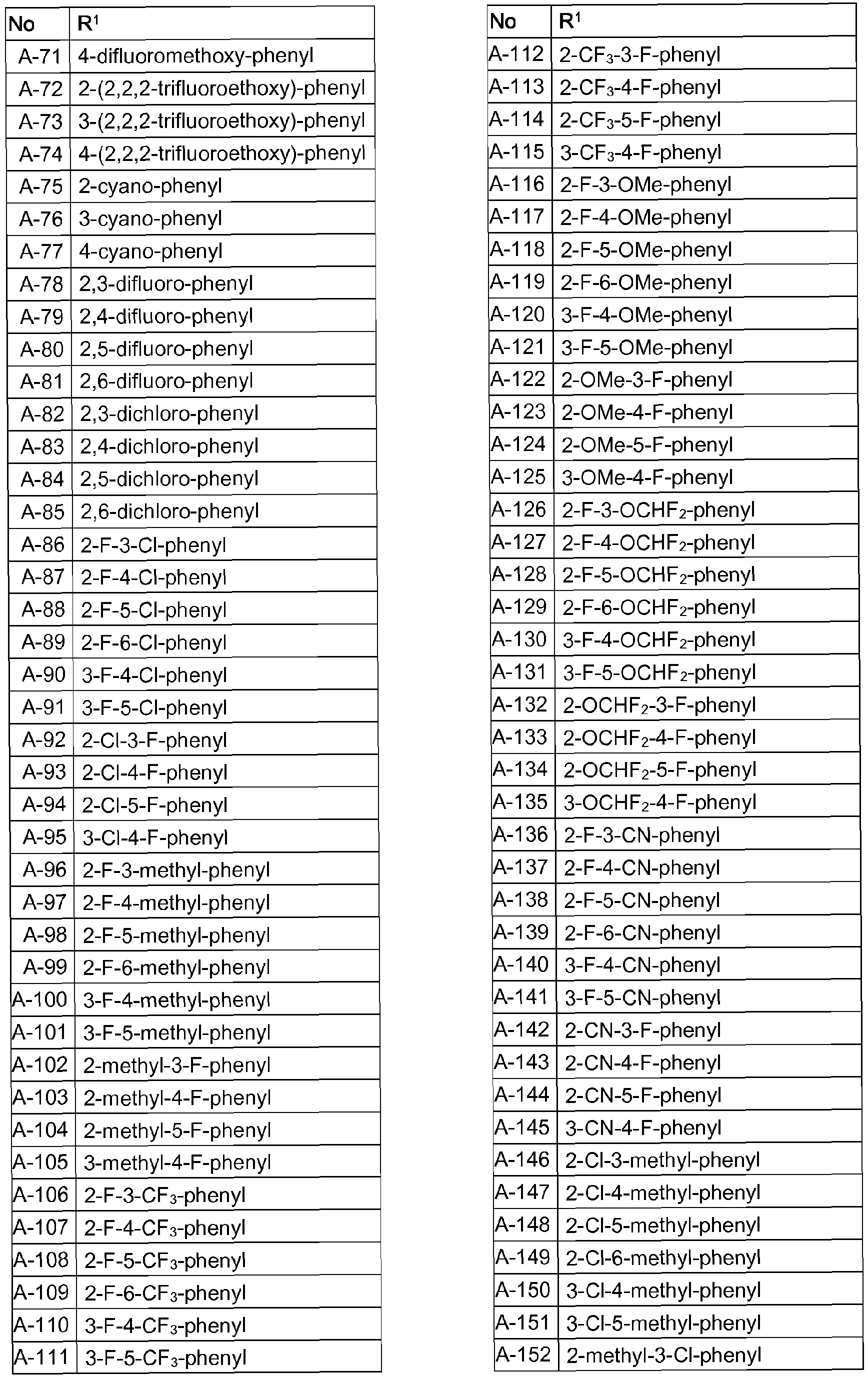

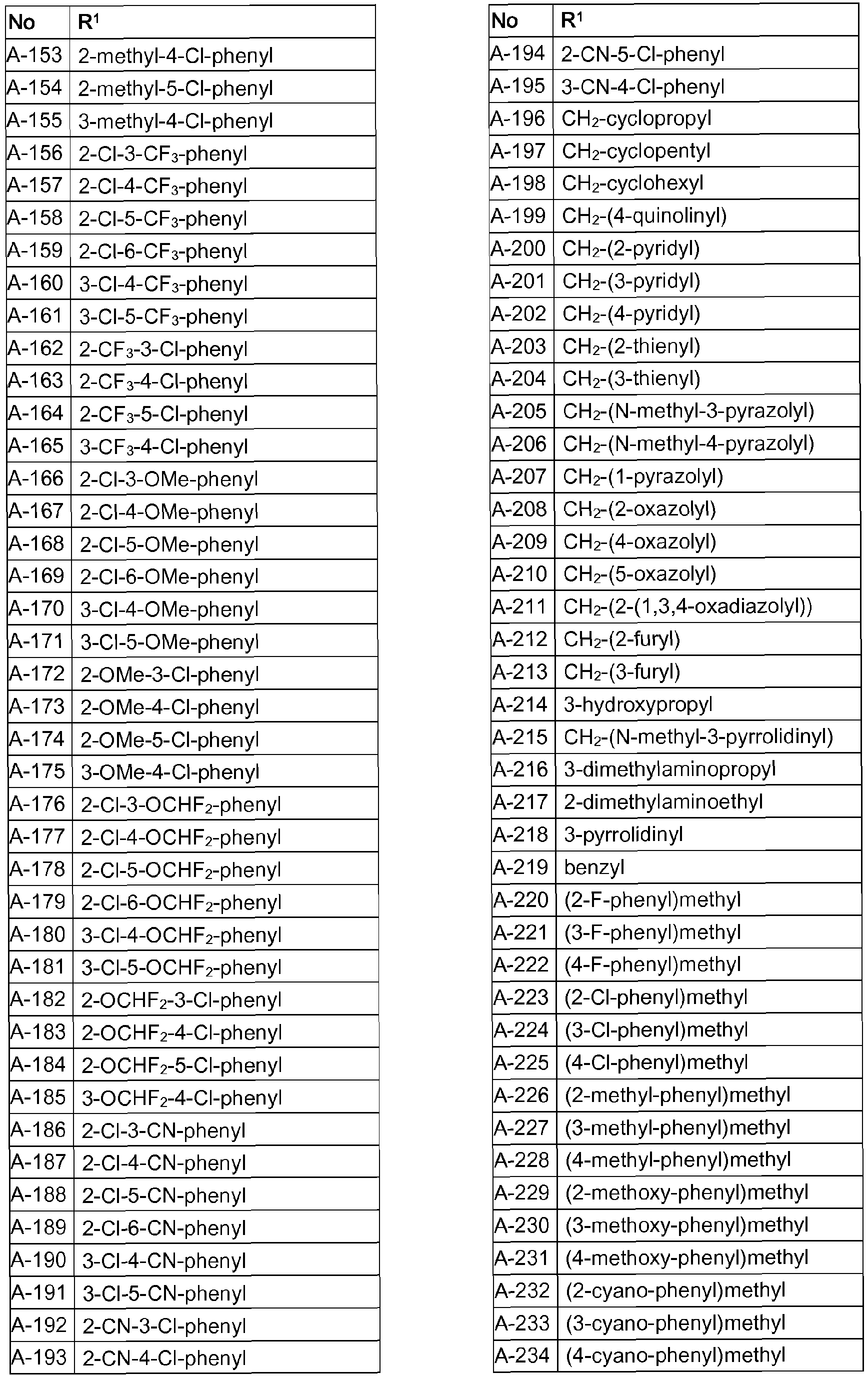

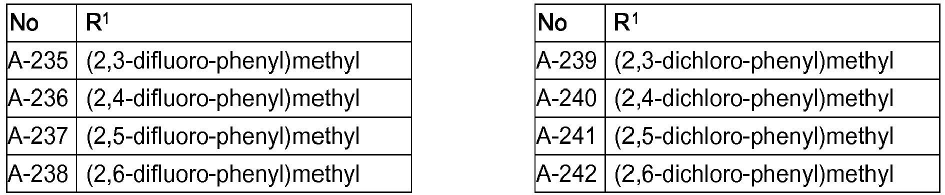

The compounds of the formula I can be prepared according to methods or in analogy to methods that are described in the prior art. The synthesis takes advantage of starting materials that are commercially available or may be prepared according to conventional procedures starting from readily available compounds.

For example, compounds of the formula I, and wherein L is -S(=O)- or -S(=O)₂- can be prepared by reacting of oxadiazole thioether of the formula II with an oxidation agent such as *m-*chloroperoxybenzoic acid or hydrogen peroxide in an organic solvent.

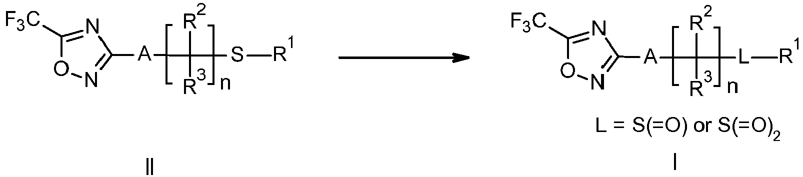

Compounds of the formula II can be prepared by reacting amidines of type III with trifluoroacetic anhydride in an organic solvent, preferably an ethereal solvent at temperatures between 0 °C and 100 °C, preferably at room temperature, as previously described in WO 2013/008162 A1.

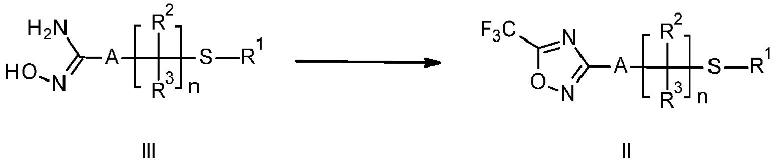

A skilled person will recognize that compounds of type III can be accessed by treating nitriles of type IV with hydroxylamine (or its HCl salt) in an organic solvent and in the presence of a base (for precedents see for example WO 2009/074950 A2, WO 2006/013104 A1, EP 1932843 A1).

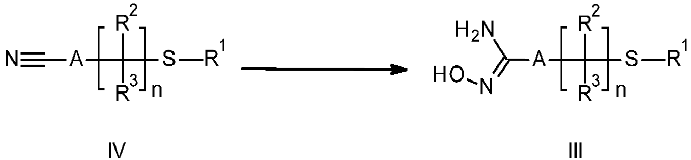

Compounds of type IV are either commercially available or can be easily accessed by reaction of a bromide of type V with the corresponding sodium thiolate in the presence of *N,N-*dimethylformamide.

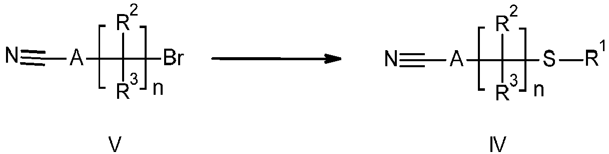

The compounds of the formula I or compositions comprising said compounds according to the invention and the mixtures comprising said compounds and compositions, respectively, are suitable as fungicides. They are distinguished by an outstanding effectiveness against a broad spectrum of phytopathogenic fungi, including soil-borne fungi, which derive especially from the following classes or are closely related to any of them: *Ascomycota (Ascomycetes),* for example, but not limited to the genus Cocholiobolus, Colletotrichum, Fusarium, Microdochium, Penicillium, Phoma, Magnaporte, Zymoseptoria, and Pseudocercosporella; *Basdiomycota (Basidiomycetes),* for example, but not limited to the genus Phakospora, Puccinia, Rhizoctonia, Sphacelotheca, Tilletia, Typhula, and Ustilago; *Chytridiomycota (Chytridiomycetes),* for example, but not limited to the genus Chytridiales, and Synchytrium; *Deuteromycetes* (syn. Fungi imperfecti), for example, but not limited to the genus *Ascochyta, Diplodia, Erysiphe, Fusarium, Phomopsis, and Pyrenophora; Peronosporomycetes* (syn. Oomycetes), for example but not limited to the genus Peronospora, Pythium, Phytophthora; *Plasmodiophoromycetes,* for example but not limited to the genus Plasmodiophora; *Zygomycetes,* for example, but not limited to the genus Rhizopus.

Some of the compounds of the formula I and the compositions according to the invention are systemically effective and they can be used in crop protection as foliar fungicides, fungicides for seed dressing and soil fungicides. Moreover, they are suitable for controlling harmful fungi, which inter alia occur in wood or roots of plants.

The compounds I and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants. Preferably, compounds I and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds I and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://cera-gmc.org/, see GM crop database therein). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties. Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxygenase (HPPD) inhibitors or phytoene desaturase (PDS) inhibitors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield^{®} summer rape (Canola, BASF SE, Germany) being tolerant to imidazolinones, e. g. imazamox, or ExpressSun^{®} sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady^{®} (glyphosate-tolerant, Monsanto, U.S.A.), Cultivance^{®} (imidazolinone tolerant, BASF SE, Germany) and LibertyLink^{®} (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieIdGard^{®} (corn cultivars producing the Cry1Ab toxin), YieldGard^{®} Plus (corn cultivars producing Cry1Ab and Cry3Bb1 toxins), Starlink^{®} (corn cultivars producing the Cry9c toxin), Herculex^{®} RW (corn cultivars producing Cry34Ab1, Cry35Ab1 and the enzyme phosphinothricin-N-acetyltransferase [PAT]); NuCOTN^{®} 33B (cotton cultivars producing the Cry1Ac toxin), Bollgard^{®} I (cotton cultivars producing the Cry1Ac toxin), Bollgard^{®} II (cotton cultivars producing Cry1Ac and Cry2Ab2 toxins); VIPCOT^{®} (cotton cultivars producing a VIP-toxin); NewLeaf^{®} (potato cultivars producing the Cry3A toxin); Bt-Xtra^{®}, NatureGard^{®}, KnockOut^{®}, BiteGard^{®}, Protecta^{®}, Bt11 (e. g. Agrisure^{®} CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1F toxin and PAT enzyme). Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the Mexican wild potato *Solanum bulbocastanum*) or T4-lysozym (e. g. potato cultivars capable of synthesizing these proteins with increased resistance against bacteria such as *Erwinia amylvora*). The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera^{®} rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora^{®} potato, BASF SE, Germany).

The compounds I and compositions thereof, respectively, are particularly suitable for controlling the following plant diseases:
*Albugo* spp. (white rust) on ornamentals, vegetables (e. g. *A. candida*) and sunflowers (e. g. *A. tragopogonis*); *Alternaria* spp. (Alternaria leaf spot) on vegetables, rape (*A. brassicola* or *brassicae*), sugar beets (*A. tenuis*), fruits, rice, soybeans, potatoes (e. g. *A. solani or A. alternata*), tomatoes (e. g. *A. solani* or *A. alternata)* and wheat; *Aphanomyces* spp. on sugar beets and vegetables; *Ascochyta* spp. on cereals and vegetables, e. g. *A. tritici* (anthracnose) on wheat and *A. hordei on* barley; *Bipolaris* and *Drechslera* spp. (teleomorph: *Cochliobolus* spp.), e. g. Southern leaf blight (*D. maydis*) or Northern leaf blight (*B. zeicola*) on corn, e. g. spot blotch (*B. sorokiniana*) on cereals and e. g. *B. oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckelianar* grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C. ulmi* (Dutch elm disease) on elms; *Cercospora* spp. (Cercospora leaf spots) on corn (e. g. Gray leaf spot: *C. zeae-maydis),* rice, sugar beets (e. g. *C. beticola*), sugar cane, vegetables, coffee, soybeans (e. g. *C. sojina or C*. *kikuchii*) and rice; *Cladosporium* spp. on tomatoes (e. g. *C. fulvum:* leaf mold) and cereals, e. g. *C. herbarum* (black ear) on wheat; *Claviceps purpurea* (ergot) on cereals; *Cochliobolus* (anamorph: *Helminthosporium* of *Bipolaris*) spp. (leaf spots) on corn (*C*. *carbonum*), cereals (e. g. *C. sativus,* anamorph: *B. sorokiniana*) and rice (e. g. *C. miyabeanus,* anamorph: *H*. *oryzae*); *Colletotrichum* (teleomorph: *Glomerella*) spp. (anthracnose) on cotton (e. g. *C*. *gossypii*), corn (e. g. *C. graminicola:* Anthracnose stalk rot), soft fruits, potatoes (e. g. *C*. *coccodes:* black dot), beans (e. g. *C. lindemuthianum*) and soybeans (e. g. *C. truncatum* or *C*. *gloeosporioides*); *Corticium* spp., e. g. *C. sasakii* (sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri*: Black Foot Disease) and ornamentals; *Dematophora* (teleomorph: *Rosellinia*) *necatrix(root* and stem rot) on soybeans; *Diaporthe* spp., e. g. *D. phaseolorum* (damping off) on soybeans; *Drechslera* (syn. *Helminthosporium,* teleomorph: *Pyrenophora*) spp. on corn, cereals, such as barley (e. g. *D. teres,* net blotch) and wheat (e. g. *D. tritici-repentis:* tan spot), rice and turf; Esca (dieback, apoplexy) on vines, caused by *Formitiporia* (syn. *Phellinus*) *punctata, F. mediterranea, Phaeomoniella chlamydospora* (earlier *Phaeoacremonium chlamydosporum*), *Phaeoacremonium aleophilum* and/or *Botryosphaeria obtusa; Elsinoe* spp. on pome fruits (*E*. *pyri*), soft fruits (*E. veneta*: anthracnose) and vines (*E. ampelina*: anthracnose); *Entyloma oryzae* (leaf smut) on rice; *Epicoccum spp.* (black mold) on wheat; *Erysiphe* spp. (powdery mildew) on sugar beets (*E. betae*), vegetables (e. g. *E. pisi*), such as cucurbits (e. g. *E. cichoracearum*), cabbages, rape (e. g. *E. cruciferarum*); *Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella*) spp. (wilt, root or stem rot) on various plants, such *as F. graminearum* or *F. culmorum* (root rot, scab or head blight) on cereals (e. g. wheat or *barley*), *F. oxysporum* on *tomatoes, F. solani* (f. sp. *glycines* now syn. *F*. *virguliforme*) and *F. tucumaniae* and *F. brasiliense* each causing sudden death syndrome on soybeans, and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi*: Bakanae disease); *Glomerella cingulata* on vines, pome fruits and other plants and *G. gossypiion* cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals and rice; *Hemileia* spp., e. g. *H. vastatrix* (coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici,* Septoria blotch) on wheat or *M*. *fijiensis* (black Sigatoka disease) on bananas; *Peronospora* spp. (downy mildew) on cabbage (e. g. *P. brassicae*), rape (e. g. *P. parasitica*), onions (e. g. *P. destructor*), tobacco (*P. tabacina*) and soybeans (e. g. *P. manshurica*); *Phakopsora pachyrhizi* and *P*. *meibomiae* (soybean rust) on soybeans; *Phialophora* spp. e.g. on vines (e. g. *P. tracheiphila* and *P. tetraspora*) and soybeans (e. g. *P. gregata*: stem rot); *Phoma lingam* (root and stem rot) on rape and cabbage and *P. betae* (root rot, leaf spot and damping-off) on sugar beets; *Phomopsis* spp*.* on sunflowers, vines (e. g. *P. viticola:* can and leaf spot) and soybeans (e. g. stem rot: *P. phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*), soybeans (e. g. *P. megasperma,* syn. *P. sojae*)*,* potatoes and tomatoes (e. g. *P. infestans*: late blight) and broad-leaved trees (e. g. *P. ramorum*: sudden oak death); *Plasmodiophora brassicae* (club root) on cabbage, rape, radish and other plants; *Plasmopara* spp., e. g. *P. viticola* (grapevine downy mildew) on vines and *P. halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat (*P. graminis*) and sugar beets (*P. betae*) and thereby transmitted viral diseases; *Pseudocercosporella herpotrichoides* (eyespot, teleomorph: *Tapesia yallundae*) on cereals, e. g. wheat or barley; *Pseudoperonospora* (downy mildew) on various plants, e. g. *P. cubensis* on cucurbits or *P. humili* on hop; *Pseudopezicula tracheiphila* (red fire disease or ,rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e. g. wheat, barley or rye, *P. kuehnii* (orange rust) on sugar cane and *P. asparagi* on asparagus; *Pyrenophora* (anamorph: *Drechslera*) *tritici-repentis* (tan spot) on wheat or *P. teres* (net blotch) on barley; *Pyricularia* spp., e. g. *P. oryzae* (teleomorph: *Magnaporthe grisea,* rice blast) on rice and *P. grisea* on turf and cereals; *Pythium* spp. (damping-off) on turf, rice, corn, wheat, cotton, rape, sunflowers, soybeans, sugar beets, vegetables and various other plants (e. g. *P. ultimum* or *P. aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R. cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sarocladium oryzae* and *S. attenuatum* (sheath rot) on rice; *Sclerotinia* spp. (stem rot or white mold) on vegetables and field crops, such as rape, sunflowers (e. g. *S. sclerotiorum*) and soybeans (e. g. *S. rolfsii or S. sclerotiorum*); *Septoria* spp. on various plants, e. g. *S. glycines* (brown spot) on soybeans, *S. tritici* (Septoria blotch) on wheat and *S.* (syn. *Stagonospora*) nodorum (Stagonospora blotch) on cereals; *Uncinula* (syn. *Erysiphe*) *necator*(powdery mildew, anamorph: *Oidium tuckeri*) on vines; *Setospaeria* spp. (leaf blight) on corn (e. g. *S. turcicum,* syn. *Helminthosporium turcicum*) and turf; *Sphacelotheca* spp. (smut) on corn, (e.g. *S*. *reiliana*: head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. *S. nodorum* (Stagonospora blotch, teleomorph: *Leptosphaeria* [syn. *Phaeosphaeria*] *nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn. *Chalara elegans*); *Tilletia* spp. (common bunt or stinking smut) on cereals, such as e. g. *T. tritici* (syn. *T. caries,* wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoli*) and sugar beets (e. g. *U. betae*); *Ustilago* spp. (loose smut) on cereals (e. g. *U. nuda* and *U. avaenae*), corn (e. g. *U. maydis:* corn smut) and sugar cane; *Venturia* spp. (scab) on apples (e. g. *V. inaequalis*) and pears; and *Verticillium* spp. (wilt) on various plants, such as fruits and ornamentals, vines, soft fruits, vegetables and field crops, e. g. *V. dahliae* on strawberries, rape, potatoes and tomatoes. In a preferred embodiment the compounds I and compositions thereof, respectively, are particularly suitable for controlling the following plant diseases: *Pucciniaspp.* (rusts) on various plants, for example, but not limited to *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e. g. wheat, barley or rye and *Phakopsoraceae* spp. on various plants, in particular *Phakopsora pachyrhizi* and *P. meibomiae* (soybean rust) on soybeans. The compounds I and compositions thereof, respectively, are also suitable for controlling harmful fungi in the protection of stored products or harvest and in the protection of materials. The term "protection of materials" is to be understood to denote the protection of technical and non-living materials, such as adhesives, glues, wood, paper and paperboard, textiles, leather, paint dispersions, plastics, cooling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus spp.;* Basidiomycetes such as *Coniophora spp., Coriolus* spp., *Gloeophyllum spp., Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichoderma* spp., *Alternaria* spp., *Paecilomyces* spp. and Zygomycetes such as *Mucor* spp., and in addition in the protection of stored products and harvest the following yeast fungi are worthy of note: *Candida* spp. and *Saccharomyces cerevisae.* The method of treatment according to the invention can also be used in the field of protecting stored products or harvest against attack of fungi and microorganisms. According to the present invention, the term "stored products" is understood to denote natural substances of plant or animal origin and their processed forms, which have been taken from the natural life cycle and for which long-term protection is desired. Stored products of crop plant origin, such as plants or parts thereof, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and the like. The combinations according the present invention can prevent disadvantageous effects such as decay, discoloration or mold. Preferably "stored products" is understood to denote natural substances of plant origin and their processed forms, more preferably fruits and their processed forms, such as pomes, stone fruits, soft fruits and citrus fruits and their processed forms.

The compounds of formula I can be present in different crystal modifications whose biological activity may differ. They are likewise subject matter of the present invention.

The compounds I are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds I as such or a composition comprising at least one compound I prophylactically either at or before planting or transplanting. The invention also relates to agrochemical compositions comprising an auxiliary and at least one compound I according to the invention.

An agrochemical composition comprises a fungicidally effective amount of a compound I. The term "effective amount" denotes an amount of the composition or of the compounds I, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials such as seeds (e. g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e. g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e. g. ethanol, propanol, butanol, benzyl alcohol, cyclohexanol; glycols; DMSO; ketones, e. g. cyclohexanone; esters, e. g. lactates, carbonates, fatty acid esters, gamma-butyrolactone; fatty acids; phosphonates; amines; amides, e. g. N-methyl pyrrolidone, fatty acid dimethyl amides; and mixtures thereof. Suitable solid carriers or fillers are mineral earths, e. g. silicates, silica gels, talc, kaolins, limestone, lime, chalk, clays, dolomite, diatomaceous earth, bentonite, calcium sulfate, magnesium sulfate, magnesium oxide; polysaccharides, e. g. cellulose, starch; fertilizers, e. g. ammonium sulfate, ammonium phosphate, ammonium nitrate, ureas; products of vegetable origin, e. g. cereal meal, tree bark meal, wood meal, nutshell meal, and mixtures thereof. Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkyl naphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the compound I on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e. g. xanthan gum, carboxymethyl cellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids. Suitable colorants (e. g. in red, blue, or green) are pigments of low water solubility and water-soluble dyes. Examples are inorganic colorants (e. g. iron oxide, titan oxide, iron hexacyanoferrate) and organic colorants (e. g. alizarin-, azo- and phthalocyanine colorants). Suitable tackifiers or binders are polyvinyl pyrrolidones, polyvinyl acetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I and 5-15 wt% wetting agent (e. g. alcohol alkoxylates) are dissolved in water and/or in a water-soluble solvent (e. g. alcohols) ad 100 wt%. The active substance dissolves upon dilution with water.
ii) Dispersible concentrates (DC)
   5-25 wt% of a compound I and 1-10 wt% dispersant (e. g. polyvinyl pyrrolidone) are dissolved in organic solvent (e. g. cyclohexanone) ad 100 wt%. Dilution with water gives a dispersion.
iii) Emulsifiable concentrates (EC)
   15-70 wt% of a compound I and 5-10 wt% emulsifiers (e. g. calcium dodecylbenzenesulfonate and castor oil ethoxylate) are dissolved in water-insoluble organic solvent (e. g. aromatic hydrocarbon) ad 100 wt%. Dilution with water gives an emulsion.
iv) Emulsions (EW, EO, ES)
   5-40 wt% of a compound I and 1-10 wt% emulsifiers (e. g. calcium dodecylbenzenesulfonate and castor oil ethoxylate) are dissolved in 20-40 wt% water-insoluble organic solvent (e. g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound I are comminuted with addition of 2-10 wt% dispersants and wetting agents (e. g. sodium lignosulfonate and alcohol ethoxylate), 0.1-2 wt% thickener (e. g. xanthan gum) and water ad 100 wt% to give a fine active substance suspension. Dilution with water gives a stable suspension of the active substance. For FS type composition up to 40 wt% binder (e. g. polyvinyl alcohol) is added.
vi) Water-dispersible granules and water-soluble granules (WG, SG)
   50-80 wt% of a compound I are ground finely with addition of dispersants and wetting agents (e. g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I are ground in a rotor-stator mill with addition of 1-5 wt% dispersants (e. g. sodium lignosulfonate), 1-3 wt% wetting agents (e. g. alcohol ethoxylate) and solid carrier (e. g. silica gel) ad 100 wt%. Dilution with water gives a stable dispersion or solution of the active substance.
viii) Gel (GW, GF)
   In an agitated ball mill, 5-25 wt% of a compound I are comminuted with addition of 3-10 wt% dispersants (e. g. sodium lignosulfonate), 1-5 wt% thickener (e. g. carboxymethyl cellulose) and water ad 100 wt% to give a fine suspension of the active substance. Dilution with water gives a stable suspension of the active substance.
ix) Microemulsion (ME)
   5-20 wt% of a compound I are added to 5-30 wt% organic solvent blend (e. g. fatty acid dimethyl amide and cyclohexanone), 10-25 wt% surfactant blend (e. g. alcohol ethoxylate and arylphenol ethoxylate), and water ad 100 %. This mixture is stirred for 1 h to produce spontaneously a thermodynamically stable microemulsion.
x) Microcapsules (CS)
   An oil phase comprising 5-50 wt% of a compound I, 0-40 wt% water insoluble organic solvent (e. g. aromatic hydrocarbon), 2-15 wt% acrylic monomers (e. g. methylmethacrylate, methacrylic acid and a di- or triacrylate) are dispersed into an aqueous solution of a protective colloid (e. g. polyvinyl alcohol). Radical polymerization results in the formation of poly(meth)acrylate microcapsules. Alternatively, an oil phase comprising 5-50 wt% of a compound I according to the invention, 0-40 wt% water insoluble organic solvent (e. g. aromatic hydrocarbon), and an isocyanate monomer (e. g. diphenylmethene-4,4'-diisocyanatae) are dispersed into an aqueous solution of a protective colloid (e. g. polyvinyl alcohol). The addition of a polyamine (e. g. hexamethylenediamine) results in the formation of polyurea microcapsules. The monomers amount to 1-10 wt%. The wt% relate to the total CS composition.
xi) Dustable powders (DP, DS)
   1-10 wt% of a compound I are ground finely and mixed intimately with solid carrier (e. g. finely divided kaolin) ad 100 wt%.
xii) Granules (GR, FG)
   0.5-30 wt% of a compound I is ground finely and associated with solid carrier (e. g. silicate) ad 100 wt%. Granulation is achieved by extrusion, spray-drying or fluidized bed.
xiii) Ultra-low volume liquids (UL)
   1-50 wt% of a compound I are dissolved in organic solvent (e. g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, more preferably between 1 and 70%, and in particular between 10 and 60%, by weight of active substance. The active substances are employed in a purity of from 90% to 100%, preferably from 95% to 100% (according to NMR spectrum).
For the purposes of treatment of plant propagation materials, particularly seeds, solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and compositions thereof, respectively, onto plant propagation material, especially seeds, include dressing, coating, pelleting, dusting, and soaking as well as in-furrow application methods. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha. In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e. g. herbicides, insecticides, fungicides, growth regulators, safeners, biopesticides) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

A pesticide is generally a chemical or biological agent (such as pestidal active ingredient, compound, composition, virus, bacterium, antimicrobial or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term "pesticide" includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology e.g. to increase plant growth, biomass, yield or any other quality parameter of the harvestable goods of a crop plant.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank or any other kind of vessel used for applications (e. g. seed treater drums, seed pelleting machinery, knapsack sprayer) and further auxiliaries may be added, if appropriate.

Consequently, one embodiment of the invention is a kit for preparing a usable pesticidal composition, the kit comprising a) a composition comprising component 1) as defined herein and at least one auxiliary; and b) a composition comprising component 2) as defined herein and at least one auxiliary; and optionally c) a composition comprising at least one auxiliary and optionally a further active component 3) as defined herein.

Mixing the compounds I or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of pesticides II (e. g. pesticidally-active substances and biopesticides), in conjunction with which the compounds I can be used, is intended to illustrate the possible combinations but does not limit them:
A) Respiration inhibitors: Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoximmethyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17), 2-(2-(3-(2,6-dichlorophenyl)-1-methyl-allylideneaminooxymethyl)-phenyl)-2-methoxyimino-N-methyl-acetamide (A.1.18), pyribencarb (A.1.19), triclopyricarb/chlorodincarb (A.1.20), famoxadone (A.1.21), fenamidone (A.1.21), methyl-*N*-[2-[(1,4-dimethyl-5-phenyl-pyrazol-3-yl)oxylmethyl]phenyl]-N-methoxy-carbamate (A.1.22), 1-[3-chloro-2-[[1-(4-chlorophenyl)-1H-pyrazol-3-yl]oxymethyl]phenyl]-4-methyl-tetrazol-5-one (A.1.23), 1-[3-bromo-2-[[1-(4-chlorophenyl)pyrazol-3-yl]oxymethyl]phenyl]-4-methyl-tetrazol-5-one (A.1.24), 1-[2-[[1-(4-chlorophenyl)pyrazol-3-yl]oxymethyl]-3-methyl-phenyl]-4-methyl-tetrazol-5-one (A.1.25), 1-[2-[[1-(4-chlorophenyl)pyrazol-3-yl]oxymethyl]-3-fluoro-phenyl]-4-methyl-tetrazol-5-one (A.1.26), 1-[2-[[1-(2,4-dichlorophenyl)pyrazol-3-yl]oxymethyl]-3-fluoro-phenyl]-4-methyl-tetrazol-5-one (A.1.27), 1-[2-[[4-(4-chlorophenyl)thiazol-2-yl]oxymethyl]-3-methyl-phenyl]-4-methyl-tetrazol-5-one (A.1.28), 1-[3-chloro-2-[[4-(p-tolyl)thiazol-2-yl]oxymethyl]phenyl]-4-methyl-tetrazol-5-one (A.1.29), 1-[3-cyclopropyl-2-[[2-methyl-4-(1-methylpyrazol-3-yl)phenoxy]-methyl]phenyl]-4-methyl-tetrazol-5-one (A.1.30), 1-[3-(difluoromethoxy)-2-[[2-methyl-4-(1-methylpyrazol-3-yl)phenoxy]methyl]phenyl]-4-methyl-tetrazol-5-one (A.1.31), 1-methyl-4-[3-methyl-2-[[2-methyl-4-(1-methylpyrazol-3-yl)phenoxy]methyl]phenyl]tetrazol-5-one (A.1.32), 1-methyl-4-[3-methyl-2-[[1-[3-(trifluoromethyl)phenyl]-ethylideneamino]oxymethyl]phenyl]tetrazol-5-one (A.1.33), (*Z*,2*E*)-5-[1-(2,4-dichlorophenyl)pyrazol-3-yl]-oxy-2-methoxyimino-*N*,3-dimethyl-pent-3-enamide (A.1.34), (*Z*,2*E*)-5-[1-(4-chlorophenyl)pyrazol-3-yl]oxy-2-methoxyimino-*N*,3-dimethyl-pent-3-enamide (A.1.35), pyriminostrobin (A.1.36), bifujunzhi (A.1.37), 2-(ortho-((2,5-dimethylphenyl-oxymethylen)phenyl)-3-methoxy-acrylic acid methylester (A.1.38). Inhibitors of complex III at Qᵢ site: cyazofamid (A.2.1), amisulbrom (A.2.2), [(6S,7R,8R)-8-benzyl-3-[(3-hydroxy-4-methoxy-pyridine-2-carbonyl)amino]-6-methyl-4,9-dioxo-1,5-dioxonan-7-yl] 2-methylpropanoate (A.2.3), fenpicoxamid (A.2.4), [(6S,7R,8R)-8-benzyl-3-[[4-methoxy-3-(propanoyloxymethoxy)pyridine-2-carbonyl]amino]-6-methyl-4,9-dioxo-1,5-dioxonan-7-yl] 2-methylpropanoate (A.2.5).
   Inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), 3-(difluoromethyl)-N-methoxy-1-methyl-N-[1-methyl-2-(2,4,6-trichlorophenyl)ethyl]pyrazole-4-carboxamide (A.3.17), N-[2-(3,4-difluorophenyl)phenyl]-3-(trifluoromethyl)pyrazine-2-carboxamide (A.3.18), sedaxane (A.3.19), tecloftalam (A.3.20), thifluzamide (A.3.21), 3-(difluoromethyl)-1-methyl-N-(1,1,3-trimethylindan-4-yl)pyrazole-4-carboxamide (A.3.22), 3-(trifluoromethyl)-1-methyl-N-(1,1,3-trimethylindan-4-yl)pyrazole-4-carboxamide (A.3.23), 1,3-dimethyl-N-(1,1,3-trimethylindan-4-yl)pyrazole-4-carboxamide (A.3.24), 3-(trifluoromethyl)-1,5-dimethyl-N-(1,1,3-trimethylindan-4-yl)pyrazole-4-carboxamide (A.3.25), 1,3,5-trimethyl-N-(1,1,3-trimethylindan-4-yl)pyrazole-4-carboxamide (A.3.26), 3-(difluoromethyl)-1,5-dimethyl-N-(1,1,3-trimethylindan-4-yl)pyrazole-4-carboxamide (A.3.27), 3-(difluoromethyl)-N-(7-fluoro-1,1,3-trimethyl-indan-4-yl)-1-methyl-pyrazole-4-carboxamide (A.3.28), methyl (E)-2-[2-[(5-cyano-2-methyl-phenoxy)methyl]phenyl]-3-methoxy-prop-2-enoate (A.3.30), N-[(5-chloro-2-isopropyl-phenyl)methyl]-N-cyclopropyl-3-(difluoromethyl)-5 fluoro-1-methyl-pyrazole-4-carboxamide (A.3.31), 2-(difluoromethyl)-N-(1,1,3-trimethyl-indan-4-yl)pyridine-3-carboxamide (A.3.32), 2-(difluoromethyl)-N-[(3R)-1,1,3-trimethylindan-4-yl]pyridine-3-carboxamide (A.3.33), 2-(difluoromethyl)-N-(3-ethyl-1,1-dimethyl-indan-4-yl)¬pyridine-3-carboxamide (A.3.34), 2-(difluoromethyl)-N-[(3R)-3-ethyl-1,1-dimethyl-indan-4-yl]¬pyridine-3-carboxamide (A.3.35), 2-(difluoromethyl)-N-(1,1-dimethyl-3-propyl-indan-4-yl)¬py¬ridine-3-carboxamide (A.3.36), 2-(difluoromethyl)-N-[(3R)-1,1-dimethyl-3-propyl-indan-4-yl]¬pyridine-3-carboxamide (A.3.37), 2-(difluoromethyl)-N-(3-isobutyl-1,1-dimethyl-indan-4-yl)¬pyridine-3-carboxamide (A.3.38), 2-(difluoromethyl)-N-[(3R)-3-isobutyl-1,1-dimethyl-indan-4 yl]pyridine-3-carboxamide (A.3.39).
   Other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12).
B) Sterol biosynthesis inhibitors (SBI fungicides)
   C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 1-[*rel*-(2*S*;3*R*)-3-(2-chlorophenyl)-2-(2,4-difluorophenyl)-oxiranylmethyl]-5-thiocyanato-1H-[1,2,4]triazole (B.1.31), 2-[*rel*-(2*S*,3*R*)-3-(2-chlorophenyl)-2-(2,4-difluorophenyl)-oxiranylmethyl]-2H-[1,2,4]triazole-3-thiol (B.1.32), 2-[2-chloro-4-(4-chlorophenoxy)phenyl]-1-(1,2,4-triazol-1-yl)pentan-2-ol (B.1.33), 1-[4-(4-chlorophenoxy)-2-(trifluoromethyl)phenyl]-1-cyclopropyl-2-(1,2,4-triazol-1-yl)ethanol (B.1.34), 2-[4-(4-chlorophenoxy)-2-(trifluoromethyl)phenyl]-1-(1,2,4-triazol-1-yl)butan-2-ol (B.1.35), 2-[2-chloro-4-(4-chlorophenoxy)phenyl]-1-(1,2,4-triazol-1-yl)butan-2-ol (B.1.36), ipfentrifluconazole (B.1.37), mefentrifluconazole (B.1.38), 2-[2-chloro-4-(4-chlorophenoxy)phenyl]-3-methyl-1-(1,2,4-triazol-1-yl)butan-2-ol (B.1.39), 2-[4-(4-chlorophenoxy)-2-(trifluoromethyl)phenyl]-1-(1,2,4-triazol-1-yl)pentan-2-ol (B.1.40), 2-[4-(4-fluorophenoxy)-2-(trifluoromethyl)phenyl]-1-(1,2,4-triazol-1-yl)propan-2-ol (B.1.41), 2-[2-chloro-4-(4-chlorophenoxy)phenyl]-1-(1,2,4-triazol-1-yl)pent-3-yn-2-ol (B.1.42), 2-(chloromethyl)-2-methyl-5-(p-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines and piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52).
   Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8).
   Inhibitors of 3-keto reductase: fenhexamid (B.3.1).
   Other Sterol biosynthesis inhibitors: chlorphenomizole (B.4.1).
C) Nucleic acid synthesis inhibitors
   Phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7). Other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8).
D) Inhibitors of cell division and cytoskeleton
   Tubulin inhibitors: benomyl (D.1.1), carbendazim (D.1.2), fuberidazole (D1.3), thiabendazole (D.1.4), thiophanate-methyl (D.1.5), 3-chloro-4-(2,6-difluorophenyl)-6-methyl-5-phenyl-pyridazine (D.1.6), 3-chloro-6-methyl-5-phenyl-4-(2,4,6-trifluorophenyl)pyridazine (D.1.7), N-ethyl-2-[(3-ethynyl-8-methyl-6-quinolyl)oxy]butanamide (D.1.8), N-ethyl-2-[(3-ethynyl-8-methyl-6-quinolyl)oxy]-2-methylsulfanyl-acetamide (D.1.9), 2-[(3-ethynyl-8-methyl-6-quinolyl)oxy]-N-(2-fluoroethyl)butanamide (D.1.10), 2-[(3-ethynyl-8-methyl-6-quinolyl)oxy]-N-(2-fluoroethyl)-2-methoxy-acetamide (D.1.11), 2-[(3-ethynyl-8-methyl-6-quinolyl)oxy]-N-propyl-butanamide (D.1.12), 2-[(3-ethynyl-8-methyl-6-quinolyl)oxy]-2-methoxy-N-propyl-acetamide (D.1.13), 2-[(3-ethynyl-8-methyl-6-quinolyl)oxy]-2-methylsulfanyl-N-propyl-acetamide (D.1.14), 2-[(3-ethynyl-8-methyl-6-quinolyl)oxy]-N-(2-fluoroethyl)-2-methylsulfanyl-acetamide (D.1.15), 4-(2-bromo-4-fluoro-phenyl)-N-(2-chloro-6-fluoro-phenyl)-2,5-dimethyl-pyrazol-3-amine (D.1.16). Other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7).
E) Inhibitors of amino acid and protein synthesis
   Methionine synthesis inhibitors: cyprodinil (E.1.1), mepanipyrim (E.1.2), pyrimethanil (E.1.3). Protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6).
F) Signal transduction inhibitors
   MAP / histidine kinase inhibitors: fluoroimid (F.1.1), iprodione (F.1.2), procymidone (F.1.3), vinclozolin (F.1.4), fludioxonil (F.1.5).
   G protein inhibitors: quinoxyfen (F.2.1).
G) Lipid and membrane synthesis inhibitors
   Phospholipid biosynthesis inhibitors: edifenphos (G.1.1), iprobenfos (G.1.2), pyrazophos (G.1.3), isoprothiolane (G.1.4).
   Lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7).
   Phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7).
   Compounds affecting cell membrane permeability and fatty acides: propamocarb (G.4.1). Inhibitors of oxysterol binding protein: oxathiapiprolin (G.5.1), 2-{3-[2-(1-{[3,5-bis(difluoromethyl-1H-pyrazol-1-yl]acetyl}piperidin-4-yl)-1,3-thiazol-4-yl]-4,5-dihydro-1,2-oxazol-5-yl}phenyl methanesulfonate (G.5.2), 2-{3-[2-(1-{[3,5-bis(difluoromethyl)-1H-pyrazol-1-yl]acetyl}piperidin-4-yl) 1,3-thiazol-4-yl]-4,5-dihydro-1,2-oxazol-5-yl}-3-chlorophenyl methanesulfonate (G.5.3), 4-[1-[2-[3-(difluoromethyl)-5-methyl-pyrazol-1-yl]acetyl]-4-piperidyl]-N-tetralin-1-yl-pyridine-2-carboxamide (G.5.4), 4-[1-[2-[3,5-bis(difluoromethyl)pyrazol-1-yl]acetyl]-4-piperidyl]-N-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-N-tetralin-1-yl-pyridine-2-carboxamide (G.5.6), 4-[1-[2-[5-cyclopropyl-3-(difluoromethyl)pyrazol-1-yl]acetyl]-4-piperidyl]-N-tetralin-1-yl-pyridine-2-carboxamide (G.5.7), 4-[1-[2-[5-methyl-3-(trifluoromethyl)pyrazol-1-yl]acetyl]-4-piperidyl]-N-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-N-tetralin-1-yl-pyridine-2-carboxamide (G.5.9), 4-[1-[2-[3,5-bis(trifluoromethyl)pyrazol-1-yl]acetyl]-4-piperidyl]-N-tetralin-1-yl-pyridine-2-carboxamide (G.5.10), (4-[1-[2-[5-cyclopropyl-3-(trifluoromethyl)pyrazol-1-yl]acetyl]-4-piperidyl]-N-tetralin-1-yl-pyridine-2-carboxamide (G.5.11).
H) Inhibitors with Multi Site Action
   Inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7).
   Thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9).
   Organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11).
   Guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1H,5H-[1,4]dithiino[2,3-c:5,6-c']dipyrrole-1,3,5,7(2H,6H)-tetraone (H.4.10).
I) Cell wall synthesis inhibitors
   Inhibitors of glucan synthesis: validamycin (I.1.1), polyoxin B (I.1.2).
   Melanin synthesis inhibitors: pyroquilon (I.2.1), tricyclazole (I.2.2), carpropamid (I.2.3), dicyclomet (I.2.4), fenoxanil (I.2.5).
J) Plant defence inducers
   Acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), potassium or sodium bicarbonate (J.1.9), 4-cyclopropyl-N-(2,4-dimethoxyphenyl)thiadiazole-5-carboxamide (J.1.10), calcium phosphonate (J.1.11), potassium phosphonate (J.1.12).
K) Unknown mode of action
   Bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flusulfamide (K.1.15), flutianil (K.1.16), harpin (K.1.17), methasulfocarb (K.1.18), nitrapyrin (K.1.19), nitrothal-isopropyl (K.1.20), tolprocarb (K.1.21), oxincopper (K.1.22), proquinazid (K.1.23), tebufloquin (K.1.24), tecloftalam (K.1.25), triazoxide (K.1.26), N'-(4-(4-chloro-3-trifluoromethyl-phenoxy)-2,5-dimethyl-phenyl)-N-ethyl-N-methyl formamidine (K.1.27), N'-(4-(4-fluoro-3-trifluoromethyl-phenoxy)-2,5-dimethyl-phenyl)-N-ethyl-N-methyl formamidine (K.1.28), N'-[4-[[3-[(4-chlorophenyl)methyl]-1,2,4-thiadiazol-5-yl]oxy]-2,5-dimethyl-phenyl]-N-ethyl-N-methyl-formamidine (K.1.29), N'-(5-bromo-6-indan-2-yloxy-2-methyl-3-pyridyl)-N-ethyl-N-methyl-formamidine (K.1.30), N'-[5-bromo-6-[1-(3,5-difluorophenyl)ethoxy]-2-methyl-3-pyridyl]-N-ethyl-N-methyl-formamidine (K.1.31), N'-[5-bromo-6-(4-isopropylcyclohexoxy)-2-methyl-3-pyridyl]-N-ethyl-N-methyl-formamidine (K.1.32), N'-[5-bromo-2-methyl-6-(1-phenylethoxy)-3-pyridyl]-N-ethyl-N-methyl-formamidine (K.1.33), N'-(2-methyl-5-trifluoromethyl-4-(3-trimethylsilanyl-propoxy)-phenyl)-N-ethyl-N-methyl formamidine (K.1.34), N'-(5-difluoromethyl-2-methyl-4-(3-trimethylsilanyl-propoxy)-phenyl)-N-ethyl-N-methyl formamidine (K.1.35), 2-(4-chloro-phenyl)-N-[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3 yl]-pyridine (K.1.38), 5-chloro-1-(4,6-dimethoxy-pyrimidin-2-yl)-2-methyl-1H-benzoimidazole (K.1.39), ethyl (Z)-3-amino-2-cyano-3-phenyl-prop-2-enoate (K.1.40), picarbutrazox (K.1.41), pentyl N-[6-[[(Z)-[(1-methyltetrazol-5-yl)-phenyl-methylene]amino]oxymethyl]-2-pyridyl]carbamate (K.1.42), but-3-ynyl N-[6-[[(Z)-[(1-methyltetrazol-5-yl)-phenyl-methylene]amino]oxymethyl]-2-pyridyl]carbamate (K.1.43), 2-[2-[(7,8-difluoro-2-methyl-3-quinolyl)oxy]-6-fluoro-phenyl]propan-2-ol (K.1.44), 2-[2-fluoro-6-[(8-fluoro-2-methyl-3-quinolyl)oxy]phen-yl]propan-2-ol (K.1.45), 3-(5-fluoro-3,3,4,4-tetramethyl-3,4-dihydroisoquinolin-1-yl)quinoline (K.1.46), quinofumelin (K.1.47), 3-(4,4,5-trifluoro-3,3-dimethyl-3,4-dihydroisoquinolin-1-yl)quinoline (K.1.48), 9-fluoro-2,2-dimethyl-5-(3-quinolyl)-3H-1,4-benzoxazepine (K.1.49), 2-(6-benzyl-2-pyridyl)quinazoline (K.1.50), 2-[6-(3-fluoro-4-methoxy-phenyl)-5-methyl-2-pyridyl]quinazoline (K.1.51), dichlobentiazox (K.1.52), N'-(2,5-dimethyl-4-phenoxy-phenyl)-N-ethyl-N-methyl-formamidine (K.1.53).
M) Growth regulators
   abscisic acid (M.1.1), amidochlor, ancymidol, 6-benzylaminopurine, brassinolide, butralin, chlormequat, chlormequat chloride, choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid , maleic hydrazide, mefluidide, mepiquat, mepiquat chloride, naphthaleneacetic acid, N-6-benzyladenine, paclobutrazol, prohexadione, prohexadione-calcium, prohydrojasmon, thidiazuron, triapenthenol, tributyl phosphorotrithioate, 2,3,5-tri-iodobenzoic acid , trinexapac-ethyl and uniconazole;
N) Herbicides from classes N.1 to N.15
   N.1 Lipid biosynthesis inhibitors: alloxydim (N.1.1), alloxydim-sodium (N.1.2), butroxydim (N.1.3), clethodim (N.1.4), clodinafop (N.1.5), clodinafop-propargyl (N.1.6), cycloxydim (N.1.7), cyhalofop (N.1.8), cyhalofop-butyl (N.1.9), diclofop(N.1.10), diclofop-methyl (N.1.11), fenoxaprop (N.1.12), fenoxaprop-ethyl (N.1.13), fenoxaprop-P (N.1.14), fenoxaprop-P-ethyl (N.1.15), fluazifop (N.1.16), fluazifop-butyl (N.1.17), fluazifop-P (N.1.18), fluazifop-P-butyl (N.1.19), haloxyfop (N.1.20), haloxyfop-methyl (N.1.21), haloxyfop-P (N.1.22), haloxyfop-P-methyl (N.1.23), metamifop (N.1.24), pinoxaden (N.1.25), profoxydim (N.1.26), propaquizafop (N.1.27), quizalofop (N.1.28), quizalofop-ethyl (N.1.29), quizalofop-tefuryl (N.1.30), quizalofop-P (N.1.31), quizalofop-P-ethyl (N.1.32), quizalofop-P-tefuryl (N.1.33), sethoxydim (N.1.34), tepraloxydim (N.1.35), tralkoxydim (N.1.36), 4-(4'-chloro-4-cyclo¬propyl-2'-fluoro[1,1'-biphenyl]-3-yl)-5-hydroxy-2,2,6,6-tetramethyl-2H-pyran-3(6H)-one ((N.1.37) CAS 1312337-72-6); 4-(2',4'-dichloro-4-cyclopropyl[1,1'-biphenyl]-3-yl)-5-hydroxy-2,2,6,6-tetramethyl-2H-pyran-3(6H)-one ((N.1.38) CAS 1312337-45-3); 4-(4'-chloro-4-ethyl-2'-fluoro[1,1'-biphenyl]-3-yl)-5-hydroxy-2,2,6,6-tetramethyl-2H-pyran-3(6H)-one ((N.1.39) CAS 1033757-93-5); 4-(2',4'-Dichloro-4-ethyl[1,1'-biphenyl]-3-yl)-2,2,6,6-tetramethyl-2H-pyran-3,5(4H,6H)-dione ((N.1.40) CAS 1312340-84-3); 5-(acetyloxy)-4-(4'-chloro-4-cyclopropyl-2'-fluoro[1,1'-biphenyl]-3-yl)-3,6-dihydro-2,2,6,6-tetramethyl-2H-pyran-3-one ((N.1.41) CAS 1312337-48-6); 5-(acetyloxy)-4-(2',4'-dichloro-4-cyclopropyl- [1,1'-biphenyl]-3-yl)-3,6-dihydro-2,2,6,6-tetramethyl-2H-pyran-3-one (N.1.42); 5-(acetyloxy)-4-(4'-chloro-4-ethyl-2'-fluoro[1,1'-biphenyl]-3-yl)-3,6-dihydro-2,2,6,6-tetramethyl-2H-pyran-3-one ((N.1.43) CAS 1312340-82-1); 5-(acetyloxy)-4-(2',4'-dichloro-4-ethyl[1,1'-biphenyl]-3-yl)-3,6-dihydro-2,2,6,6-tetramethyl-2H-pyran-3-one ((N.1.44) CAS 1033760-55-2); 4-(4'-chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester ((N.1.45) CAS 1312337-51-1); 4-(2',4'-dichloro -4-cyclopropyl- [1,1'-biphenyl]-3-yl)-5,6-dihydro-2,2,6,6-tetramethyl-5-oxo-2H-pyran-3-yl carbonic acid methyl ester (N.1.46); 4-(4'-chloro-4-ethyl-2'-fluoro[1,1'-biphenyl]-3-yl)-5,6-dihydro-2,2,6,6-tetramethyl-5-oxo-2H-pyran-3-yl carbonic acid methyl ester ((N.1.47) CAS 1312340-83-2); 4-(2',4'-dichloro-4-ethyl¬[1,1'-biphenyl]-3-yl)-5,6-dihydro-2,2,6,6-tetramethyl-5-oxo-2H-pyran-3-yl carbonic acid methyl ester ((N.1.48) CAS 1033760-58-5); benfuresate (N.1.49), butylate (N.1.50), cycloate (N.1.51), dalapon (N.1.52), dimepiperate (N.1.53), EPTC (N.1.54), esprocarb (N.1.55), ethofumesate (N.1.56), flupropanate (N.1.57), molinate (N.1.58), orbencarb (N.1.59), pebulate (N.1.60), prosulfocarb (N.1.61), TCA (N.1.62), thiobencarb (N.1.63), tiocarbazil (N.1.64), triallate (N.1.65) and vernolate (N.1.66);
   N.2 ALS inhibitors: amidosulfuron (N.2.1), azimsulfuron (N.2.2), bensulfuron (N.2.3), bensulfuron-methyl (N.2.4), chlorimuron (N.2.5), chlorimuron-ethyl (N.2.6), chlorsulfuron (N.2.7), cinosulfuron (N.2.8), cyclosulfamuron (N.2.9), ethametsulfuron (N.2.10), ethametsulfuron-methyl (N.2.11), ethoxysulfuron (N.2.12), flazasulfuron (N.2.13), flucetosulfuron (N.2.14), flupyrsulfuron (N.2.15), flupyrsulfuron-methyl-sodium (N.2.16), foramsulfuron (N.2.17), halosulfuron (N.2.18), halosulfuron-methyl (N.2.19), imazosulfuron (N.2.20), iodosulfuron (N.2.21), iodosulfuron-methyl-sodium (N.2.22), iofensulfuron (N.2.23), iofensulfuron-sodium (N.2.24), mesosulfuron (N.2.25), metazosulfuron (N.2.26), metsulfuron (N.2.27), metsulfuron-methyl (N.2.28), nicosulfuron (N.2.29), orthosulfamuron (N.2.30), oxasulfuron (N.2.31), primisulfuron (N.2.32), primisulfuron-methyl (N.2.33), propyrisulfuron (N.2.34), prosulfuron (N.2.35), pyrazosulfuron (N.2.36), pyrazosulfuron-ethyl (N.2.37), rimsulfuron (N.2.38), sulfometuron (N.2.39), sulfometuron-methyl (N.2.40), sulfosulfuron (N.2.41), thifensulfuron (N.2.42), thifensulfuron-methyl (N.2.43), triasulfuron (N.2.44), tribenuron (N.2.45), tribenuron-methyl (N.2.46), trifloxysulfuron (N.2.47), triflusulfuron (N.2.48), triflusulfuron-methyl (N.2.49), tritosulfuron (N.2.50), imazamethabenz (N.2.51), imazamethabenz-methyl (N.2.52), imazamox (N.2.53), imazapic (N.2.54), imazapyr (N.2.55), imazaquin (N.2.56), imazethapyr (N.2.57); cloransulam (N.2.58), cloransulam-methyl (N.2.59), diclosulam (N.2.60), flumetsulam (N.2.61), florasulam (N.2.62), metosulam (N.2.63), penoxsulam (N.2.64), pyrimisulfan (N.2.65) and pyroxsulam (N.2.66); bispyribac (N.2.67), bispyribac-sodium (N.2.68), pyribenzoxim (N.2.69), pyriftalid (N.2.70), pyriminobac (N.2.71), pyriminobac-methyl (N.2.72), pyrithiobac (N.2.73), pyrithiobac-sodium (N.2.74), 4-[[[2-[(4,6-dimethoxy-2-pyrimidinyl)oxy]phenyl]methyl]amino]-benzoic acid-1-methyl¬ethyl ester ((N.2.75) CAS 420138-41-6), 4-[[[2-[(4,6-dimethoxy-2-pyrimidinyl)oxy]phenyl]¬methyl]amino]-benzoic acid propyl ester ((N.2.76) CAS 420138-40-5), N-(4-bromophenyl)-2-[(4,6-dimethoxy-2-pyrimidinyl)oxy]benzenemethanamine ((N.2.77) CAS 420138-01-8); flucarbazone (N.2.78), flucarbazone-sodium (N.2.79), propoxycarbazone (N.2.80), propoxycarbazone-sodium (N.2.81), thiencarbazone (N.2.82), thiencarbazone-methyl (N.2.83), triafamone (N.2.84);
   N.3 Photosynthesis inhibitors: amicarbazone (N.3.1); chlorotriazine (N.3.2); ametryn (N.3.3), atrazine (N.3.4), chloridazone (N.3.5), cyanazine (N.3.6), desmetryn (N.3.7), dimethametryn (N.3.8),hexazinone (N.3.9), metribuzin (N.3.10), prometon (N.3.11), prometryn (N.3.12), pro-pazine (N.3.13), simazine (N.3.14), simetryn (N.3.15), terbumeton (N.3.16), terbuthylazin (N.3.17), terbutryn (N.3.18), trietazin (N.3.19); chlorobromuron (N.3.20), chlorotoluron (N.3.21), chloroxuron (N.3.22), dimefuron (N.3.23), diuron (N.3.24), fluometuron (N.3.25), isoproturon (N.3.26), isouron (N.3.27), linuron (N.3.28), metamitron (N.3.29), methabenzthiazuron (N.3.30), metobenzuron (N.3.31), metoxuron (N.3.32), monolinuron (N.3.33), neburon (N.3.34), siduron (N.3.35), tebuthiuron (N.3.36), thiadiazuron (N.3.37), desmedipham (N.3.38), karbutilat (N.3.39), phenmedipham (N.3.40), phenmedipham-ethyl (N.3.41), bromofenoxim (N.3.42), bromoxynil (N.3.43) and its salts and esters, ioxynil (N.3.44) and its salts and esters, bromacil (N.3.45), lenacil (N.3.46), terbacil (N.3.47), bentazon (N.3.48), bentazon-sodium (N.3.49), pyridate (N.3.50), pyridafol (N.3.51), pentanochlor (N.3.52), propanil (N.3.53); diquat (N.3.54), diquatdibromide (N.3.55), paraquat (N.3.56), paraquat-dichloride (N.3.57), paraquat-dimetilsulfate (N.3.58);
   N.4 protoporphyrinogen-IX oxidase inhibitors: acifluorfen (N.4.1), acifluorfen-sodium (N.4.2), azafenidin (N.4.3), bencarbazone (N.4.4), benzfendizone (N.4.5), bifenox (N.4.6), butafenacil (N.4.7), carfentrazone (N.4.8), carfentrazone-ethyl (N.4.9), chlormethoxyfen (N.4.10), cinidon-ethyl (N.4.11), fluazolate (N.4.12), flufenpyr (N.4.13), flufenpyr-ethyl (N.4.14), flumiclorac (N.4.15), flumiclorac-pentyl (N.4.16), flumioxazin (N.4.17), fluoroglycofen (N.4.18), fluoroglycofen-ethyl (N.4.19), fluthiacet (N.4.20), fluthiacet-methyl (N.4.21), fomesafen (N.4.22), halosafen (N.4.23), lactofen (N.4.24), oxadiargyl (N.4.25), oxadiazon (N.4.26), oxyfluorfen (N.4.27), pentoxazone (N.4.28), profluazol (N.4.29), pyraclonil (N.4.30), pyraflufen (N.4.31), pyraflufen-ethyl (N.4.32), saflufenacil (N.4.33), sulfentrazone (N.4.34), thidiazimin (N.4.35), tiafenacil (N.4.36), trifludimoxazin (N.4.37), ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate ((N.4.38) CAS 353292-31-6), N-ethyl-3-(2,6-dichloro-4-trifluoro-methylphenoxy)-5-methyl-1 H-pyrazole-1-carboxamide ((N.4.39) CAS 452098-92-9), N tetrahydrofurfuryl-3-(2,6-dichloro-4-trifluoromethylphenoxy)-5-methyl-1H-pyrazole-1-carboxamide ((N.4.40) CAS 915396-43-9), N-ethyl-3-(2-chloro-6-fluoro-4-trifluoromethyl¬phenoxy)-5-methyl-1H-pyrazole-1-carboxamide ((N.4.41) CAS 452099-05-7), N tetrahydro¬furfuryl-3-(2-chloro-6-fluoro-4-trifluoro¬methylphenoxy)-5-methyl-1H-pyrazole-1-carboxamide ((N.4.42) CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione ((N.4.43) CAS 451484-50-7), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione ((N.4.44) CAS 1300118-96-0), 1-methyl-6-trifluoro¬methyl-3-(2,2,7-tri-fluoro-3-oxo-4-prop-2-ynyl-3,4-dihydro-2H-benzo[1,4]oxazin-6-yl)-1H-pyrimidine-2,4-dione ((N.4.45) CAS 1304113-05-0), methyl (E)-4-[2-chloro-5-[4-chloro-5-(difluoromethoxy)-1H-methyl-pyrazol-3-yl]-4-fluoro-phenoxy]-3-methoxy-but-2-enoate ((N.4.46) CAS 948893-00-3), 3-[7-chloro-5-fluoro-2-(trifluoromethyl)-1H-benzimidazol-4-yl]-1-methyl-6-(trifluoromethyl)-1 H-pyrimidine-2,4-dione ((N.4.47) CAS 212754-02-4);
   N.5 Bleacher herbicides: beflubutamid (N.5.1), diflufenican (N.5.2), fluridone (N.5.3), flurochloridone (N.5.4), flurtamone (N.5.5), norflurazon (N.5.6), picolinafen (N.5.7), 4-(3-trifluoromethyl¬phenoxy)-2-(4-trifluoromethylphenyl)¬pyrimidine ((N.5.8) CAS 180608-33-7); benzobicyclon (N.5.9), benzofenap (N.5.10), bicyclopyrone (N.5.11), clomazone (N.5.12), fenquintrione (N.5.13), isoxaflutole (N.5.14), mesotrione (N.5.15), pyrasulfotole (N.5.16), pyrazolynate (N.5.17), pyrazoxyfen (N.5.18), sulcotrione (N.5.19), tefuryltrione (N.5.20), tembotrione (N.5.21), tolpyralate (N.5.22), topramezone (N.5.23); aclonifen (N.5.24), amitrole (N.5.25), flumeturon (N.5.26);
   N.6 EPSP synthase inhibitors: glyphosate (N.6.1), glyphosate-isopropylammonium (N.6.2), glyposate-potassium (N.6.3), glyphosate-trimesium (sulfosate) (N.6.4);
   N.7 Glutamine synthase inhibitors: bilanaphos (bialaphos) (N.7.1), bilanaphos-sodium (N.7.2), glufosinate (N.7.3), glufosinate-P (N.7.4), glufosinate-ammonium (N.7.5);
   N.8 DHP synthase inhibitors: asulam (N.8.1);
   N.9 Mitosis inhibitors: benfluralin (N.9.1), butralin (N.9.2), dinitramine (N.9.3), ethalfluralin (N.9.4), fluchloralin (N.9.5), oryzalin (N.9.6), pendimethalin (N.9.7), prodiamine (N.9.8), trifluralin (N.9.9); amiprophos (N.9.10), amiprophos-methyl (N.9.11), butamiphos (N.9.12); chlorthal (N.9.13), chlorthal-dimethyl (N.9.14), dithiopyr (N.9.15), thiazopyr (N.9.16), propyzamide (N.9.17), tebutam (N.9.18); carbetamide (N.9.19), chlorpropham (N.9.20), flamprop (N.9.21), flamprop-isopropyl (N.9.22), flamprop-methyl (N.9.23), flamprop-M-isopropyl (N.9.24), flamprop-M-methyl (N.9.25), propham (N.9.26);
   N.10 VLCFA inhibitors: acetochlor (N.10.1), alachlor (N.10.2), butachlor (N.10.3), dimethachlor (N.10.4), dimethenamid (N.10.5), dimethenamid-P (N.10.6), metazachlor (N.10.7), metolachlor (N.10.8), metolachlor-S (N.10.9), pethoxamid (N.10.10), pretilachlor (N.10.11), propachlor (N.10.12), propisochlor (N.10.13), thenylchlor (N.10.14), flufenacet (N.10.15), mefenacet (N.10.16), diphenamid (N.10.17), naproanilide (N.10.18), napropamide (N.10.19), napropamide-M (N.10.20), fentrazamide (N.10.21), anilofos (N.10.22), cafenstrole (N.10.23), fenoxasulfone (N.10.24), ipfencarbazone (N.10.25), piperophos (N.10.26), pyroxasulfone (N.10.27), isoxazoline compounds of the formulae II.1, II.2, II.3, II.4, II.5, II.6, II.7, II.8 and II.9 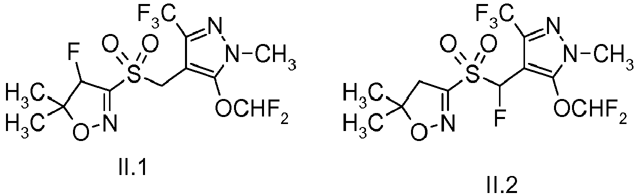 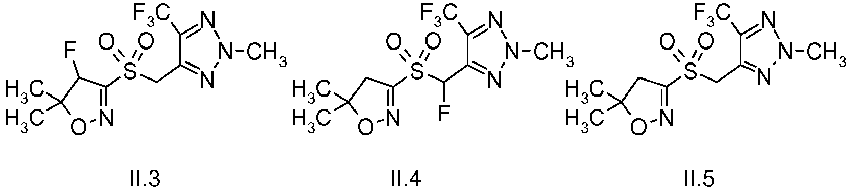 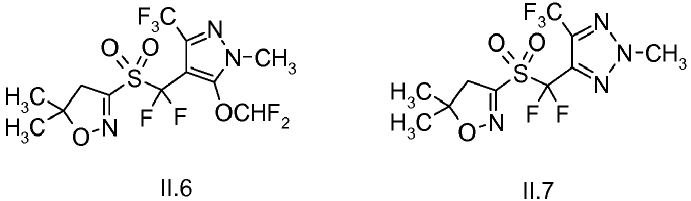 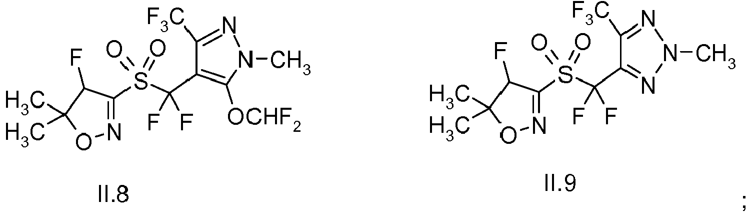
   N.11 Cellulose biosynthesis inhibitors: chlorthiamid (N.11.1), dichlobenil (N.11.2), flupoxam (N.11.3), indaziflam (N.11.4), isoxaben (N.11.5), triaziflam (N.11.6), 1-cyclohexyl-5-pentafluorphenyloxy-14-[1,2,4,6]thiatriazin-3-ylamine ((N.11.7) CAS 175899-01-1);
   N.12 Decoupler herbicides: dinoseb (N.12.1), dinoterb (N.12.2), DNOC (N.12.3) and its salts;
   N.13 Auxinic herbicides: 2,4-D (N.13.1) and its salts and esters, clacyfos (N.13.2), 2,4-DB (N.13.3) and its salts and esters, aminocyclopyrachlor (N.13.4) and its salts and esters, aminopyralid (N.13.5) and its salts such as aminopyralid-dimethylammonium (N.13.6), aminopyralid-tris(2-hydroxypropyl)ammonium (N.13.7) and its esters, benazolin (N.13.8), benazolin-ethyl (N.13.9), chloramben (N.13.10) and its salts and esters, clomeprop (N.13.11), clopyralid (N.13.12) and its salts and esters, dicamba (N.13.13) and its salts and esters, dichlorprop (N.13.14) and its salts and esters, dichlorprop-P (N.13.15) and its salts and esters, fluroxypyr (N.13.16), fluroxypyr-butometyl (N.13.17), fluroxypyr-meptyl (N.13.18), halauxifen (N.13.) and its salts and esters (CAS 943832-60-8); MCPA (N.13.) and its salts and esters, MCPA-thioethyl (N.13.19), MCPB (N.13.20) and its salts and esters, mecoprop (N.13.21) and its salts and esters, mecoprop-P (N.13.22) and its salts and esters, picloram (N.13.23) and its salts and esters, quinclorac (N.13.24), quinmerac (N.13.25), TBA (2,3,6) (N.13.26) and its salts and esters, triclopyr (N.13.27) and its salts and esters, 4-amino-3-chloro-6-(4-chloro-2-fluoro-3-methoxyphenyl)-5-fluoropyridine-2-carboxylic acid (N.13.28), benzyl 4-amino-3-chloro-6-(4-chloro-2-fluoro-3-methoxyphenyl)-5-fluoropyridine-2-carboxylate ((N.13.29) CAS 1390661-72-9);
   N.14 Auxin transport inhibitors: diflufenzopyr (N.14.1), diflufenzopyr-sodium (N.14.2), naptalam (N.14.3) and naptalam-sodium (N.14.4);
   N.15 Other herbicides: bromobutide (N.15.1), chlorflurenol (N.15.2), chlorflurenol-methyl (N.15.3), cinmethylin (N.15.4), cumyluron (N.15.5), cyclopyrimorate ((N.15.6) CAS 499223-49-3) and its salts and esters, dalapon (N.15.7), dazomet (N.15.8), difenzoquat (N.15.9), difenzoquat-metilsulfate (N.15.10), dimethipin (N.15.11), DSMA (N.15.12), dymron (N.15.13), endothal (N.15.14) and its salts, etobenzanid (N.15.15), flurenol (N.15.16), flurenol-butyl (N.15.17), flurprimidol (N.15.18), fosamine (N.15.19), fosamine-ammonium (N.15.20), indanofan (N.15.21), maleic hydrazide (N.15.22), mefluidide (N.15.23), metam (N.15.24), methiozolin ((N.15.25) CAS 403640-27-7), methyl azide (N.15.26), methyl bromide (N.15.27), methyl-dymron (N.15.28), methyl iodide (N.15.29), MSMA (N.15.30), oleic acid (N.15.31), oxaziclomefone (N.15.32), pelargonic acid (N.15.33), pyributicarb (N.15.34), quinoclamine (N.15.35), tridiphane (N.15.36); O) Insecticides from classes O.1 to 0.29
      O.1 Acetylcholine esterase (AChE) inhibitors: aldicarb (O.1.1), alanycarb (0.1.2), bendiocarb (0.1.3), benfuracarb (0.1.4), butocarboxim (0.1.5), butoxycarboxim (0.1.6), carbaryl (0.1.7), carbofuran (0.1.8), carbosulfan (0.1.9), ethiofencarb (O.1.10), fenobucarb (O.1.11), formetanate (0.1.12), furathiocarb (0.1.13), isoprocarb (0.1.14), methiocarb (0.1.15), methomyl (0.1.16), metolcarb (0.1.17), oxamyl (0.1.18), pirimicarb (0.1.19), propoxur (0.1.20), thiodicarb (0.1.21), thiofanox (0.1.22), trimethacarb (0.1.23), XMC (0.1.24), xylylcarb (0.1.25) and triazamate (0.1.26); acephate (0.1.27), azamethiphos (0.1.28), azinphos-ethyl (0.1.29), azinphosmethyl (0.1.30), cadusafos (0.1.31), chlorethoxyfos (0.1.32), chlorfenvinphos (0.1.33), chlormephos (0.1.34), chlorpyrifos (0.1.35), chlorpyrifos-methyl (0.1.36), coumaphos (0.1.37), cyanophos (0.1.38), demeton-S-methyl (0.1.39), diazinon (O.1.40)*,* dichlorvos/ DDVP (O.1.41), dicrotophos (O.1.42), dimethoate (O.1.43), dimethylvinphos (O.1.44), disulfoton (O.1.45), EPN (O.1.46), ethion (O.1.47), ethoprophos (O.1.48), famphur (O.1.49), fenamiphos (0.1.50), fenitrothion (0.1.51), fenthion (0.1.52), fosthiazate (0.1.53), heptenophos (0.1.54), imicyafos (0.1.55), isofenphos (0.1.56), isopropyl O-(methoxyaminothio-phosphoryl) salicylate (0.1.57), isoxathion (0.1.58), malathion (0.1.59), mecarbam (0.1.60), methamidophos (0.1.61), methidathion (0.1.62), mevinphos (0.1.63), monocrotophos (0.1.64), naled (0.1.65), omethoate (0.1.66), oxydemeton-methyl (0.1.67), parathion (0.1.68), parathion-methyl (0.1.69), phenthoate (0.1.70), phorate (0.1.71), phosalone (0.1.72), phosmet (0.1.73), phosphamidon (0.1.74), phoxim (0.1.75), pirimiphos- methyl (0.1.76), profenofos (0.1.77), propetamphos (0.1.78), prothiofos (0.1.79), pyraclofos (0.1.80), pyridaphenthion (0.1.81), quinalphos (0.1.82), sulfotep (0.1.83), tebupirimfos (0.1.84), temephos (0.1.85), terbufos (0.1.86), tetrachlorvinphos (0.1.87), thiometon (0.1.88), triazophos (0.1.89), trichlorfon (0.1.90), vamidothion (0.1.91);
      0.2 GABA-gated chloride channel antagonists: endosulfan (0.2.1), chlordane (0.2.2); ethiprole (0.2.3), fipronil (O.2.4), flufiprole (0.2.5), pyrafluprole (0.2.6), pyriprole (0.2.7);
      0.3 Sodium channel modulators: acrinathrin (0.3.1), allethrin (0.3.2), d-cis-trans allethrin (0.3.3), d-trans allethrin (O.3.4), bifenthrin (0.3.5), bioallethrin (0.3.6), bioallethrin S-cylclopentenyl (0.3.7), bioresmethrin (0.3.8), cycloprothrin (0.3.9), cyfluthrin (0.3.10), beta-cyfluthrin (0.3.11), cyhalothrin (0.3.12), lambda-cyhalothrin (0.3.13), gamma-cyhalothrin (0.3.14), cypermethrin (0.3.15), alpha-cypermethrin (0.3.16), beta-cypermethrin (0.3.17), theta-cypermethrin (0.3.18), zeta-cypermethrin (0.3.19), cyphenothrin (0.3.20), deltamethrin (0.3.21), empenthrin (0.3.22), esfenvalerate (0.3.23), etofenprox (0.3.24), fenpropathrin (0.3.25), fenvalerate (0.3.26), flucythrinate (0.3.27), flumethrin (0.3.28), tau-fluvalinate (0.3.29), halfenprox (0.3.30), heptafluthrin (0.3.31), imiprothrin (0.3.32), meperfluthrin (0.3.33), metofluthrin (0.3.34), momfluorothrin (0.3.35), permethrin (0.3.36), phenothrin (0.3.37), prallethrin (0.3.38), profluthrin (0.3.39), pyrethrin (pyrethrum) (O.3.40), resmethrin (O.3.41), silafluofen (O.3.42), tefluthrin (O.3.43), tetramethylfluthrin (O.3.44), tetramethrin (O.3.45), tralomethrin (O.3.46) and transfluthrin (O.3.47); DDT (O.3.48), methoxychlor (O.3.49); O.4 Nicotinic acetylcholine receptor agonists (nAChR): acetamiprid (O.4.1), clothianidin (O.4.2), cycloxaprid (O.4.3), dinotefuran (O.4.4), imidacloprid (O.4.5), nitenpyram (O.4.6), thiacloprid (O.4.7), thiamethoxam (O.4.8); (2E)-1-[(6-chloropyridin-3-yl)methyl]-N'-nitro-2-pentylidene-hydrazinecarboximidamide (O.4.9); 1-[(6-chloropyridin-3-yl)methyl]-7-methyl-8-nitro-5-propoxy-1,2,3,5,6,7-hexahydroimidazo[1,2-a]pyridine (O.4.10); nicotine (O.4.11); 0.5 Nicotinic acetylcholine receptor allosteric activators: spinosad (0.5.1), spinetoram (0.5.2); 0.6 Chloride channel activators: abamectin (0.6.1), emamectin benzoate (0.6.2), ivermectin (0.6.3), lepimectin (O.6.4), milbemectin (0.6.5);
      0.7 Juvenile hormone mimics: hydroprene (0.7.1), kinoprene (0.7.2), methoprene (0.7.3); fenoxycarb (O.7.4), pyriproxyfen (0.7.5);
      0.8 miscellaneous non-specific (multi-site) inhibitors: methyl bromide (0.8.1) and other alkyl halides; chloropicrin (0.8.2), sulfuryl fluoride (0.8.3), borax (O.8.4), tartar emetic (0.8.5);
      0.9 Selective homopteran feeding blockers: pymetrozine (0.9.1), flonicamid (0.9.2); O.10 Mite growth inhibitors: clofentezine (O.10.1), hexythiazox (0.10.2), diflovidazin (0.10.3); etoxazole (O.10.4);
      O.11 Microbial disruptors of insect midgut membranes: the Bt crop proteins: Cry1Ab, Cry1Ac, Cry1Fa, Cry2Ab, mCry3A, Cry3Ab, Cry3Bb, Cry34/35Ab1;
      0.12 Inhibitors of mitochondrial ATP synthase: diafenthiuron (0.12.1); azocyclotin (0.12.2), cyhexatin (0.12.3), fenbutatin oxide (O.12.4), propargite (0.12.5), tetradifon (0.12.6);
      0.13 Uncouplers of oxidative phosphorylation via disruption of the proton gradient: chlorfenapyr (0.13.1), DNOC (0.13.2), sulfluramid (0.13.3);
      0.14 Nicotinic acetylcholine receptor (nAChR) channel blockers: bensultap (0.14.1), cartap hydrochloride (0.14.2), thiocyclam (0.14.3), thiosultap sodium (O.14.4);
      0.15 Inhibitors of the chitin biosynthesis type 0: bistrifluron (0.15.1), chlorfluazuron (0.15.2), diflubenzuron (0.15.3), flucycloxuron (O.15.4), flufenoxuron (0.15.5), hexaflumuron (0.15.6), lufenuron (0.15.7), novaluron (0.15.8), noviflumuron (0.15.9), teflubenzuron (0.15.10), triflumuron (0.15.11);
      0.16 Inhibitors of the chitin biosynthesis type 1: buprofezin (0.16.1);
      0.17 Moulting disruptors: cyromazine (0.17.1);
      0.18 Ecdyson receptor agonists: methoxyfenozide (O.18.1), tebufenozide (0.18.2), halofenozide (0.18.3), fufenozide (O.18.4), chromafenozide (0.18.5);
      0.19 Octopamin receptor agonists: amitraz (0.19.1);
      0.20 Mitochondrial complex III electron transport inhibitors: hydramethylnon (0.20.1), acequinocyl (0.20.2), fluacrypyrim (0.20.3);
      0.21 Mitochondrial complex I electron transport inhibitors: fenazaquin (0.21.1), fenpyroximate (0.21.2), pyrimidifen (0.21.3), pyridaben (O.21.4), tebufenpyrad (0.21.5), tolfenpyrad (0.21.6); rotenone (O.21.7);
      0.22 Voltage-dependent sodium channel blockers: indoxacarb (0.22.1), metaflumizone (0.22.2), 2-[2-(4-cyanophenyl)-1-[3-(trifluoromethyl)phenyl]ethylidene]-N-[4-(difluoromethoxy)phenyl]-hydrazinecarboxamide (0.22.3), N-(3-chloro-2-methylphenyl)-2-[(4-chlorophenyl)-[4-[methyl(methylsulfonyl)amino]phenyl]methylene]-hydrazinecarboxamide (O.22.4);
      0.23 Inhibitors of the of acetyl CoA carboxylase: spirodiclofen (0.23.1), spiromesifen (0.23.2), spirotetramat (0.23.3);
      0.24 Mitochondrial complex IV electron transport inhibitors: aluminium phosphide (0.24.1), calcium phosphide (0.24.2), phosphine (0.24.3), zinc phosphide (O.24.4), cyanide (0.24.5); 0.25 Mitochondrial complex II electron transport inhibitors: cyenopyrafen (0.25.1), cyflumetofen (0.25.2);
      0.26 Ryanodine receptor-modulators: flubendiamide (0.26.1), chlorantraniliprole (0.26.2), cyantraniliprole (0.26.3), cyclaniliprole (O.26.4), tetraniliprole (0.26.5); (R)-3-chloro-N1-{2-methyl-4-[1,2,2,2 -tetrafluoro-1-(trifluoromethyl)ethyl]phenyl}-N2-(1-methyl-2-methylsulfonylethyl)phthalamide (0.26.6), (S)-3-chloro-N1-{2-methyl-4-[1,2,2,2-tetrafluoro-1-(trifluoromethyl)ethyl]phenyl}-N2-(1-methyl-2-methylsulfonylethyl)phthalamide (0.26.7), methyl-2-[3,5-dibromo-2-({[3-bromo-1-(3-chloropyridin-2-yl)-1H-pyrazol-5-yl]carbonyl}amino)benzoyl]-1,2-dimethylhydrazinecarboxylate (0.26.8); N-[4,6-dichloro-2-[(diethyl-lambda-4-sulfanylidene)-carbamoyl]-phenyl]-2-(3-chloro-2-pyridyl)-5-(trifluoromethyl)pyrazole-3-carboxamide (0.26.9); N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide (0.26.10); N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide (0.26.11); N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide (0.26.12); N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide (0.26.13); N-[2-(5-amino-1,3,4-thiadiazol-2-yl)-4-chloro-6-methylphenyl]-3-bromo-1-(3-chloro-2-pyridinyl)-1H-pyrazole-5-carboxamide (0.26.14); 3-chloro-1-(3-chloro-2-pyridinyl)-N-[2,4-dichloro-6-[[(1-cyano-1-methylethyl)amino]carbonyl]phenyl]-1H-pyrazole-5-carboxamide (0.26.15); 3-bromo-N-[2,4-dichloro-6-(methylcarbamoyl)phenyl]-1-(3,5-dichloro-2-pyridyl)-1H-pyrazole-5-carboxamide (0.26.16); N-[4-chloro-2-[[(1,1-dimethylethyl)amino]carbonyl]-6-methylphenyl]-1-(3-chloro-2-pyridinyl)-3-(fluoromethoxy)-1H-pyrazole-5-carboxamide (0.26.17); cyhalodiamide (0.26.18);
      0.27. insecticidal active compounds of unknown or uncertain mode of action: afidopyropen (0.27.1), afoxolaner (0.27.2), azadirachtin (0.27.3), amidoflumet (O.27.4), benzoximate (0.27.5), bifenazate (0.27.6), broflanilide (0.27.7), bromopropylate (0.27.8), chinomethionat (0.27.9), cryolite (0.27.10), dicloromezotiaz (0.27.11), dicofol (0.27.12), flufenerim (0.27.13), flometoquin (0.27.14), fluensulfone (0.27.15), fluhexafon (0.27.16), fluopyram (0.27.17), flupyradifurone (0.27.18), fluralaner (0.27.19), metoxadiazone (0.27.20), piperonyl butoxide (0.27.21), pyflubumide (0.27.22), pyridalyl (0.27.23), pyrifluquinazon (0.27.24), sulfoxaflor (0.27.25), tioxazafen (0.27.26), triflumezopyrim (0.27.27), 11-(4-chloro-2,6-dimethylphenyl)-12-hydroxy-1,4-dioxa-9-azadispiro[4.2.4.2]-tetradec-11-en-10-one (0.27.28), 3-(4'-fluoro-2,4-dimethylbiphenyl-3-yl)-4-hydroxy-8-oxa-1-azaspiro[4.5]dec-3-en-2-one (0.27.28), 1-[2-fluoro-4-methyl-5-[(2,2,2-trifluoroethyl)sulfinyl]phenyl]-3-(trifluoromethyl)-1H-1,2,4-triazole-5-amine (0.27.29), (E/Z)-N-[1-[(6-chloro-3-pyridyl)methyl]-2-pyridylidene]-2,2,2-trifluoro-acetamide (0.27.31); (E/Z)-N-[1-[(6-chloro-5-fluoro-3-pyridyl)methyl]-2-pyridylidene]-2,2,2-trifluoroacetamide (0.27.32); (E/Z)-2,2,2-trifluoro-N-[1-[(6-fluoro-3-pyridyl)methyl]-2-pyridylidene]acetamide (0.27.33); (E/Z)-N-[1-[(6-bromo-3-pyridyl)methyl]-2-pyridylidene]-2,2,2-trifluoroacetamide (0.27.34); (E/Z)-N-[1-[1-(6-chloro-3-pyridyl)ethyl]-2-pyridylidene]-2,2,2-trifluoroacetamide (0.27.35); (E/Z)-N-[1-[(6-chloro-3-pyridyl)methyl]-2-pyridylidene]-2,2-difluoroacetamide (0.27.36); (E/Z)-2-chloro-N-[1-[(6-chloro-3-pyridyl)methyl]-2-pyridylidene]-2,2-difluoro-acetamide (0.27.37); (E/Z)-N-[1-[(2-chloropyrimidin-5-yl)methyl]-2-pyridylidene]-2,2,2-trifluoro-acetamide (0.27.38); (E/Z)-N-[1-[(6-chloro-3-pyridyl)methyl]-2-pyridylidene]-2,2,3,3,3-pentafluoro-propanamide (0.27.39); N-[1-[(6-chloro-3-pyridyl)methyl]-2-pyridylidene]-2,2,2-trifluoro-thioacetamide (O.27.40); N-[1-[(6-chloro-3-pyridyl)methyl]-2-pyridylidene]-2,2,2-trifluoro-N'-isopropyl-acetamidine (O.27.41); fluazaindolizine (O.27.42); 4-[5-(3,5-dichlorophenyl)-5-(trifluoromethyl)-4H-isoxazol-3-yl]-2-methyl-N-(1-oxothietan-3-yl)benzamide (O.27.43); fluxametamide (O.27.44); 5-[3-[2,6-dichloro-4-(3,3-dichloroallyloxy)phenoxy]propoxy]-1H-pyrazole (O.27.45); 3-(benzoylmethylamino)-N-[2-bromo-4-[1,2,2,3,3,3-hexafluoro-1-(trifluoromethyl)propyl]-6-(trifluoromethyl)phenyl]-2-fluoro-benzamide (O.27.46); 3-(benzoylmethylamino)-2-fluoro-N-[2-iodo-4-[1,2,2,2-tetrafluoro-1-(trifluoromethyl)ethyl]-6-(trifluoromethyl)phenyl]-benzamide (O.27.47); N-[3-[[[2-iodo-4-[1,2,2,2-tetrafluoro-1-(trifluoromethyl)ethyl]-6-(trifluoromethyl)phenyl]amino]carbonyl]phenyl]-N-methyl-benzamide (O.27.48); N-[3-[[[2-bromo-4-[1,2,2,2-tetrafluoro-1-(trifluoromethyl)ethyl]-6-(trifluoromethyl)phenyl]amino]carbonyl]-2-fluorophenyl]-4-fluoro-N-methyl-benzamide (0.27.49); 4-fluoro-N-[2-fluoro-3-[[[2-iodo-4-[1,2,2,2-tetrafluoro-1-(trifluoromethyl)ethyl]-6-(trifluoromethyl)phenyl]amino]carbonyl]phenyl]-N-methyl-benzamide (0.27.50); 3-fluoro-N-[2-fluoro-3-[[[2-iodo-4-[1,2,2,2-tetrafluoro-1-(trifluoromethyl)ethyl]-6(trifluoromethyl)phenyl]amino]-carbonyl]phenyl]-N-methyl-benzamide (0.27.51); 2-chloro-N-[3-[[[2-iodo-4-[1,2,2,2-tetrafluoro-1-(trifluoromethyl)ethyl]-6-(trifluoromethyl)phenyl]amino]carbonyl]phenyl]-3-pyridinecarboxamide (0.27.52); 4-cyano-N-[2-cyano-5-[[2,6-dibromo-4-[1,2,2,3,3,3-hexafluoro-1-(trifluoromethyl)propyl]phenyl]carbamoyl]phenyl]-2-methyl-benzamide (0.27.53); 4-cyano-3-[(4-cyano-2-methyl-benzoyl)amino]-N-[2,6-dichloro-4-[1,2,2,3,3,3-hexafluoro-1-(trifluoromethyl)-propyl]phenyl]-2-fluoro-benzamide (0.27.54); N-[5-[[2-chloro-6-cyano-4-[1,2,2,3,3,3-hexafluoro-1-(trifluoromethyl)propyl]phenyl]carbamoyl]-2-cyano-phenyl]-4-cyano-2-methyl-benzamide (0.27.55); N-[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide (0.27.56); N-[5-[[2-bromo-6-chloro-4-[1,2,2,3,3,3-hexafluoro-1-(trifluoromethyl)-propyl]phenyl]carbamoyl]-2-cyano-phenyl]-4-cyano-2-methyl-benzamide (0.27.57); 4-cyano-N-[2-cyano-5-[[2,6-dichloro-4-[1,2,2,3,3,3-hexafluoro-1-(trifluoromethyl)propyl]phenyl]-carbamoyl]phenyl]-2-methyl-benzamide (0.27.58); 4-cyano-N-[2-cyano-5-[[2,6-dichloro-4-[1,2,2,2-tetrafluoro-1-(trifluoromethyl)ethyl]phenyl]carbamoyl]phenyl]-2-methyl-benzamide (0.27.59); N-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide (0.27.60); 2-(1,3-dioxan-2-yl)-6-[2-(3-pyridinyl)-5-thiazolyl]-pyridine; 2-[6-[2-(5-fluoro-3-pyridinyl)-5-thiazolyl]-2-pyridinyl]-pyrimidine (0.27.61); 2-[6-[2-(3-pyridinyl)-5-thiazolyl]-2-pyridinyl]-pyrimidine (0.27.62); N-methylsulfonyl-6-[2-(3-pyridyl)thiazol-5-yl]pyridine-2-carboxamide (0.27.63); N-methylsulfonyl-6-[2-(3-pyridyl)thiazol-5-yl]pyridine-2-carboxamide (0.27.64); N-ethyl-N-[4-methyl-2-(3-pyridyl)thiazol-5-yl]-3-methylthio-propanamide (0.27.65); N-methyl-N-[4-methyl-2-(3-pyridyl)thiazol-5-yl]-3-methylthio-propanamide (0.27.66); N,2-dimethyl-N-[4-methyl-2-(3-pyridyl)thiazol-5-yl]-3-methylthio-propanamide (0.27.67); N-ethyl-2-methyl-N-[4-methyl-2-(3-pyridyl)thiazol-5-yl]-3-methylthio-propanamide (0.27.68); N-[4-chloro-2-(3-pyridyl)thiazol-5-yl]-N-ethyl-2-methyl-3-methylthio-propanamide (0.2769.); N-[4-chloro-2-(3-pyridyl)thiazol-5-yl]-N,2-dimethyl-3-methylthio-propanamide (0.27.70); N-[4-chloro-2-(3-pyridyl)thiazol-5-yl]-N-methyl-3-methylthio-propanamide (0.27.71); N-[4-chloro-2-(3-pyridyl)thiazol-5-yl]-N-ethyl-3-methylthio-propanamide (0.27.72); 1-[(6-chloro-3-pyridinyl)methyl]-1,2,3,5,6,7-hexahydro-5-methoxy-7-methyl-8-nitro-imidazo[1,2-a]pyridine (0.27.73); 1-[(6-chloropyridin-3-yl)methyl]-7-methyl-8-nitro-1,2,3,5,6,7-hexahydroimidazo[1,2-a]pyridin-5-ol (0.27.74); 1-isopropyl-N,5-dimethyl-N-pyridazin-4-yl-pyrazole-4-carboxamide (0.27.75); 1-(1,2-dimethylpropyl)-N-ethyl-5-methyl-N-pyridazin-4-yl-pyrazole-4-carboxamide (0.27.76); N,5-dimethyl-N-pyridazin-4-yl-1-(2,2,2-trifluoro-1-methyl-ethyl)pyrazole-4-carboxamide (0.27.77); 1-[1-(1-cyanocyclopropyl)ethyl]-N-ethyl-5-methyl-N-pyridazin-4-yl-pyrazole-4-carboxamide (0.27.78); N-ethyl-1-(2-fluoro-1-methyl-propyl)-5-methyl-N-pyridazin-4-yl-pyrazole-4-carboxamide (0.27.79); 1-(1,2-dimethylpropyl)-N,5-dimethyl-N-pyridazin-4-yl-pyrazole-4-carboxamide (0.27.80); 1-[1-(1-cyanocyclopropyl)ethyl]-N,5-dimethyl-N-pyridazin-4-yl-pyrazole-4-carboxamide (0.27.81); N-methyl-1-(2-fluoro-1-methyl-propyl]-5-methyl-N-pyridazin-4-yl-pyrazole-4-carboxamide (0.27.82); 1-(4,4-difluorocyclohexyl)-N-ethyl-5-methyl-N-pyridazin-4-yl-pyrazole-4-carboxamide (0.27.83); 1-(4,4-difluorocyclohexyl)-N,5-dimethyl-N-pyridazin-4-yl-pyrazole-4-carboxamide (0.27.84), N-(1-methylethyl)-2-(3-pyridinyl)-2H-indazole-4-carboxamide (0.27.85); N-cyclopropyl-2-(3-pyridinyl)-2H-indazole-4-carboxamide (0.27.86); N-cyclohexyl-2-(3-pyridinyl)-2H-indazole-4-carboxamide (0.27.87); 2-(3-pyridinyl)-N-(2,2,2-trifluoroethyl)-2H-indazole-4-carboxamide (0.27.88); 2-(3-pyridinyl)-N-[(tetrahydro-2-furanyl)methyl]-2H-indazole-5-carboxamide (0.27.89); methyl 2-[[2-(3-pyridinyl)-2H-indazol-5-yl]carbonyl]hydrazinecarboxylate (0.27.90); N-[(2,2-difluorocyclopropyl)methyl]-2-(3-pyridinyl)-2H-indazole-5-carboxamide (0.27.91); N-(2,2-difluoropropyl)-2-(3-pyridinyl)-2H-indazole-5-carboxamide (0.27.92); 2-(3-pyridinyl)-N-(2-pyrimidinylmethyl)-2H-indazole-5-carboxamide (0.27.93); N-[(5-methyl-2-pyrazinyl)methyl]-2-(3-pyridinyl)-2H-indazole-5-carboxamide (0.27.94), N-[3-chloro-1-(3-pyridyl)pyrazol-4-yl]-N-ethyl-3-(3,3,3-trifluoropropylsulfanyl)-propanamide (0.27.95); N-[3-chloro-1-(3-pyridyl)pyrazol-4-yl]-N-ethyl-3-(3,3,3-trifluoropropylsulfinyl)propanamide (0.27.96); N-[3-chloro-1-(3-pyridyl)pyrazol-4-yl]-3-[(2,2-difluorocyclopropyl)methylsulfanyl]-N-ethyl-propanamide (0.27.97); N-[3-chloro-1-(3-pyridyl)pyrazol-4-yl]-3-[(2,2-difluorocyclopropyl)methylsulfinyl]-N-ethyl-propanamide (0.27.98); sarolaner (0.27.99), lotilaner (0.27.100).

The active substances referred to as component 2, their preparation and their activity e. g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441).

The present invention furthermore relates to agrochemical compositions comprising a mixture of at least one compound I (component 1) and at least one further active substance useful for plant protection, e. g. selected from the groups A) to O) (component 2), in particular one further fungicide, e. g. one or more fungicide from the groups A) to K), as described above, and if desired one suitable solvent or solid carrier. Those mixtures are of particular interest, since many of them at the same application rate show higher efficiencies against harmful fungi. Furthermore, combating harmful fungi with a mixture of compounds I and at least one fungicide from groups A) to K), as described above, is more efficient than combating those fungi with individual compounds I or individual fungicides from groups A) to K).

By applying compounds I together with at least one active substance from groups A) to O) a synergistic effect can be obtained, i.e. more then simple addition of the individual effects is obtained (synergistic mixtures).

This can be obtained by applying the compounds I and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

When applying compound I and a pesticide II sequentially the time between both applications may vary e. g. between 2 hours to 7 days. Also a broader range is possible ranging from 0.25 hour to 30 days, preferably from 0.5 hour to 14 days, particularly from 1 hour to 7 days or from 1.5 hours to 5 days, even more preferred from 2 hours to 1 day.

In the binary mixtures and compositions according to the invention the weight ratio of the component 1) and the component 2) generally depends from the properties of the active components used, usually it is in the range of from 1:10,000 to 10,000:1, often it is in the range of from 1:100 to 100:1, regularly in the range of from 1:50 to 50:1, preferably in the range of from 1:20 to 20:1, more preferably in the range of from 1:10 to 10:1, even more preferably in the range of from 1:4 to 4:1 and in particular in the range of from 1:2 to 2:1.

According to further embodiments of the binary mixtures and compositions, the weight ratio of the component 1) and the component 2) usually is in the range of from 1000:1 to 1:1, often in the range of from 100: 1 to 1:1, regularly in the range of from 50:1 to 1:1, preferably in the range of from 20:1 to 1:1, more preferably in the range of from 10:1 to 1:1, even more preferably in the range of from 4:1 to 1:1 and in particular in the range of from 2:1 to 1:1.

According to a further embodiments of the binary mixtures and compositions, the weight ratio of the component 1) and the component 2) usually is in the range of from 1:1 to 1:1000, often in the range of from 1:1 to 1:100, regularly in the range of from 1:1 to 1:50, preferably in the range of from 1:1 to 1:20, more preferably in the range of from 1:1 to 1:10, even more preferably in the range of from 1:1 to 1:4 and in particular in the range of from 1:1 to 1:2.

In the ternary mixtures, i.e. compositions according to the invention comprising the component 1) and component 2) and a compound III (component 3), the weight ratio of component 1) and component 2) depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1, and the weight ratio of component 1) and component 3) usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1.

Any further active components are, if desired, added in a ratio of from 20:1 to 1:20 to the component 1).

These ratios are also suitable for inventive mixtures applied by seed treatment.

Preference is also given to mixtures comprising as component 2) at least one active substance selected from inhibitors of complex III at Qₒ site in group A), more preferably selected from compounds (A.1.1), (A.1.4), (A.1.8), (A.1.9), (A.1.10), (A.1.12), (A.1.13), (A.1.14), (A.1.17), (A.1.21), (A.1.24), (A.1.25), (A.1.26), (A.1.27), (A.1.30), (A.1.31), (A.1.32), (A.1.34) and (A.1.35); particularly selected from (A.1.1), (A.1.4), (A.1.8), (A.1.9), (A.1.13), (A.1.14), (A.1.17), (A.1.24), (A.1.25), (A.1.26), (A.1.27), (A.1.30), (A.1.31), (A.1.32), (A.1.34) and (A.1.35). Preference is also given to mixtures comprising as component 2) at least one active substance selected from inhibitors of complex III at Qᵢ site in group A), more preferably selected from compounds (A.2.1), (A.2.3) and (A.2.4); particularly selected from (A.2.3) and (A.2.4). Preference is also given to mixtures comprising as component 2) at least one active substance selected from inhibitors of complex II in group A), more preferably selected from compounds (A.3.2), (A.3.3), (A.3.4), (A.3.7), (A.3.9), (A.3.11), (A.3.12), (A.3.15), (A.3.16), (A.3.17), (A.3.18), (A.3.19), (A.3.20), (A.3.21), (A.3.22), (A.3.23), (A.3.24), (A.3.25), (A.3.27), (A.3.28), (A.3.29), (A.3.31), (A.3.32), (A.3.33), (A.3.34), (A.3.35), (A.3.36), (A.3.37), (A.3.38) and (A.3.39); particularly selected from (A.3.2), (A.3.3), (A.3.4), (A.3.7), (A.3.9), (A.3.12), (A.3.15), (A.3.17), (A.3.19), (A.3.22), (A.3.23), (A.3.24), (A.3.25), (A.3.27), (A.3.29), (A.3.31), (A.3.32), (A.3.33), (A.3.34), (A.3.35), (A.3.36), (A.3.37), (A.3.38) and (A.3.39).

Preference is also given to mixtures comprising as component 2) at least one active substance selected from other respiration nhibitors in group A), more preferably selected from compounds (A.4.5) and (A.4.11); in particular (A.4.11).

Preference is also given to mixtures comprising as component 2) at least one active substance selected from C14 demethylase inhibitors in group B), more preferably selected from compounds (B.1.4), (B.1.5), (B.1.8), (B.1.10), (B.1.11), (B.1.12), (B.1.13), (B.1.17), (B.1.18), (B.1.21), (B.1.22), (B.1.23), (B.1.25), (B.1.26), (B.1.29), (B.1.34), (B.1.37), (B.1.38), (B.1.43) and (B.1.46); particularly selected from (B.1.5), (B.1.8), (B.1.10), (B.1.17), (B.1.22), (B.1.23), (B.1.25), (B.1.33), (B.1.34), (B.1.37), (B.138), (B.1.43) and (B.1.46).

Preference is also given to mixtures comprising as component 2) at least one active substance selected from Delta14-reductase inhibitors in group B), more preferably selected from compounds (B.2.4), (B.2.5), (B.2.6) and (B.2.8); in particular (B.2.4).

Preference is also given to mixtures comprising as component 2) at least one active substance selected from phenylamides and acyl amino acid fungicides in group C), more preferably selected from compounds (C.1.1), (C.1.2), (C.1.4) and (C.1.5); particularly selected from (C.1.1) and (C.1.4).

Preference is also given to mixtures comprising as component 2) at least one active substance selected from other nucleic acid synthesis inhibitors in group C), more preferably selected from compounds (C.2.6),(C.2.7) and (C.2.8).

Preference is also given to mixtures comprising as component 2) at least one active substance selected from group D), more preferably selected from compounds (D.1.1), (D.1.2), (D.1.5), (D.2.4) and (D.2.6); particularly selected from (D.1.2), (D.1.5) and (D.2.6).

Preference is also given to mixtures comprising as component 2) at least one active substance selected from group E), more preferably selected from compounds (E.1.1), (E.1.3), (E.2.2) and (E.2.3); in particular (E.1.3).

Preference is also given to mixtures comprising as component 2) at least one active substance selected from group F), more preferably selected from compounds (F.1.2), (F.1.4) and (F.1.5). Preference is also given to mixtures comprising as component 2) at least one active substance selected from group G), more preferably selected from compounds (G.3.1), (G.3.3), (G.3.6), (G.5.1), (G.5.2), (G.5.3), (G.5.4), (G.5.5), G.5.6), G.5.7), (G.5.8), (G.5.9), (G.5.10) and (G.5.11); particularly selected from (G.3.1), (G.5.1), (G.5.2) and (G.5.3).

Preference is also given to mixtures comprising as component 2) at least one active substance selected from group H), more preferably selected from compounds (H.2.2), (H.2.3), (H.2.5), (H.2.7), (H.2.8), (H.3.2), (H.3.4), (H.3.5), (H.4.9) and (H.4.10); particularly selected from (H.2.2), (H.2.5), (H.3.2), (H.4.9) and (H.4.10).

Preference is also given to mixtures comprising as component 2) at least one active substance selected from group I), more preferably selected from compounds (I.2.2) and (I.2.5). Preference is also given to mixtures comprising as component 2) at least one active substance selected from group J), more preferably selected from compounds (J.1.2), (J.1.5), (J.1.8), (J.1.11) and (J.1.12); in particular (J.1.5).

Preference is also given to mixtures comprising as component 2) at least one active substance selected from group K), more preferably selected from compounds (K.1.41), (K.1.42), (K.1.44), (K.1.45), (K.1.47) and (K.1.49); particularly selected from (K.1.41), (K.1.44), (K.1.45), (K.1.47) and (K.1.49).

Accordingly, the present invention furthermore relates to mixtures comprising one compound of the formula I (component 1) and one pesticide II (component 2), wherein pesticide II is an active ingredients selected from the groups A) to O) defined above.

Further embodiments B-1 to B-729 listed in Table B below relate to mixtures comprising as active components one of the in the present specification individualized compounds of the formula I, which is selected from the group of compounds I.A.A-1 to I.A.A-242, I.B.A-1 to I.B.A-242, I.C.A-1 to I.C.A-242, I.D.A-1 to I.D.A-242, I.E.A-1 to I.E.A-242, I.F.A-1 to I.F.A-242, I.G.A-1 to I.G.A-242, I.H.A-1 to I.H.A-242, I.J.A-1 to I.J.A-242, I.K.A-1 to I.K.A-242, I.L.A-1 to I.L.A-242, I.M.A-1 to I.M.A-242, I.N.A-1 to I.N.A-242, I.O.A-1 to I.O.A-242, I.P.A-1 to I.P.A-242, I.Q.A-1 to I.Q.A-242, I.R.A-1 to I.R.A-242, I.S.A-1 to I.S.A-242, I.T.A-1 to I.T.A-242, I.U.A-1 to I.U.A-242, I.V.A-1 to I.V.A-242 and I.W.A-1 to I.W.A-242 as defined in tables 1 to 22 (component 1, a group represented by the expression "(I)") and one pesticide II selected from the groups A) to O) (component 2).

Further embodiments B-1 to B-729 listed in Table B below relate to the mixtures comprising as active components one of the in the present specification individualized compounds of the formula I, which is selected from the group of compounds Ex-1 to Ex-30 as defined in Table I below (component 1, a group represented by the expression "(I)") and one pesticide II selected from the groups A) to O) as defined herein (component 2).

Preferably, the compositions described in Table B comprise the active components in synergistically effective amounts.

### Table B:

B-1: (I)+(A.1.1), B-2: (I)+(A.1.2), B-3: (I)+(A.1.3), B-4: (I)+(A.1.4), B-5: (I)+(A.1.5), B-6: (I)+(A.1.6), B-7: (I)+(A.1.7), B-8: (I)+(A.1.8), B-9: (I)+(A.1.9), B-10: (I)+(A.1.10), B-11: (I)+(A.1.11), B-12: (I)+(A.1.12), B-13: (I)+(A.1.13), B-14: (I)+(A.1.14), B-15: (I)+(A.1.15), B-16: (I)+(A.1.16), B-17: (I)+(A.1.17), B-18: (I)+(A.1.18), B-19: (I)+(A.1.19), B-20: (I)+(A.1.20), B-21: (I)+(A.1.21), B-22: (I)+(A.1.22), B-23: (I)+(A.1.23), B-24: (I)+(A.1.24), B-25: (I)+(A.1.25), B-26: (I)+(A.1.26), B-27: (I)+(A.1.27), B-28: (I)+(A.1.30), B-29: (I)+(A.1.31), B-30: (I)+(A.1.32), B-31: (I)+(A.2.1), B-32: (I)+(A.2.2), B-33: (I)+(A.2.3), B-34: (I)+(A.2.4), B-35: (I)+(A.2.6), B-36: (I)+(A.2.7), B-37: (I)+(A.2.8), B-38: (I)+(A.3.1), B-39: (I)+(A.3.2), B-40: (I)+(A.3.3), B-41: (I)+(A.3.4), B-42: (I)+(A.3.5), B-43: (I)+(A.3.6), B-44: (I)+(A.3.7), B-45: (I)+(A.3.8), B-46: (I)+(A.3.9), B-47: (I)+(A.3.10), B-48: (I)+(A.3.11), B-49: (I)+(A.3.12), B-50: (I)+(A.3.13), B-51: (I)+(A.3.14), B-52: (I)+(A.3.15), B-53: (I)+(A.3.16), B-54: (I)+(A.3.17), B-55: (I)+(A.3.18), B-56: (I)+(A.3.19), B-57: (I)+(A.3.20), B-58: (I)+(A.3.21), B-59: (I)+(A.3.22), B-60: (I)+(A.3.23), B-61: (I)+(A.3.24), B-62: (I)+(A.3.25), B-63: (I)+(A.3.26), B-64: (I)+(A.3.27), B-65: (I)+(A.3.28), B-66: (I)+(A.3.29), B-67: (I)+(A.3.30), B-68: (I)+(A.3.31), B-69: (I)+(A.3.32), B-70: (I)+(A.3.33), B-71: (I)+(A.3.34), B-72: (I)+(A.3.35), B-73: (I)+(A.3.36), B-74: (I)+(A.3.37), B-75: (I)+(A.3.38), B-76: (I)+(A.3.39), B-77: (I)+(A.4.1), B-78: (I)+(A.4.2), B-79: (I)+(A.4.3), B-80: (I)+(A.4.4), B-81: (I)+(A.4.5), B-82: (I)+(A.4.6), B-83: (I)+(A.4.7), B-84: (I)+(A.4.8), B-85: (I)+(A.4.9), B-86: (I)+(A.4.10), B-87: (I)+(A.4.11), B-88: (I)+(A.4.12), B-89: (I)+(B.1.1), B-90: (I)+(B.1.2), B-91: (I)+(B.1.3), B-92: (I)+(B.1.4), B-93: (I)+(B.1.5), B-94: (I)+(B.1.6), B-95: (I)+(B.1.7), B-96: (I)+(B.1.8), B-97: (I)+(B.1.9), B-98: (I)+(B.1.10), B-99: (I)+(B.1.11), B-100: (I)+(B.1.12), B-101: (I)+(B.1.13), B-102: (I)+(B.1.14), B-103: (I)+(B.1.15), B-104: (I)+(B.1.16), B-105: (I)+(B.1.17), B-106: (I)+(B.1.18), B-107: (I)+(B.1.19), B-108: (I)+(B.1.20), B-109: (I)+(B.1.21), B-110: (I)+(B.1.22), B-111: (I)+(B.1.23), B-112: (I)+(B.1.24), B-113: (I)+(B.1.25), B-114: (I)+(B.1.26), B-115: (I)+(B.1.27), B-116: (I)+(B.1.28), B-117: (I)+(B.1.29), B-118: (I)+(B.1.30), B-119: (I)+(B.1.34), B-120: (I)+(B.1.37), B-121: (I)+(B.1.38), B-122: (I)+(B.1.43), B-123: (I)+(B.1.44), B-124: (I)+(B.1.45), B-125: (I)+(B.1.46), B-126: (I)+(B.1.47), B-127: (I)+(B.1.48), B-128: (I)+(B.1.49), B-129: (I)+(B.1.50), B-130: (I)+(B.1.51), B-131: (I)+(B.2.1), B-132: (I)+(B.2.2), B-133: (I)+(B.2.3), B-134: (I)+(B.2.4), B-135: (I)+(B.2.5), B-136: (I)+(B.2.6), B-137: (I)+(B.2.7), B-138: (I)+(B.2.8), B-139: (I)+(B.3.1), B-140: (I)+(C.1.1), B-141: (I)+(C.1.2), B-142: (I)+(C.1.3), B-143: (I)+(C.1.4), B-144: (I)+(C.1.5), B-145: (I)+(C.1.6), B-146: (I)+(C.1.7), B-147: (I)+(C.2.1), B-148: (I)+(C.2.2), B-149: (I)+(C.2.3), B-150: (I)+(C.2.4), B-151: (I)+(C.2.5), B-152: (I)+(C.2.6), B-153: (I)+(C.2.7), B-154: (I)+(D.1.1), B-155: (I)+(D.1.2), B-156: (I)+(D.1.3), B-157: (I)+(D.1.4), B-158: (I)+(D.1.5), B-159: (I)+(D.1.6), B-160: (I)+(D.2.1), B-161: (I)+(D.2.2), B-162: (I)+(D.2.3), B-163: (I)+(D.2.4), B-164: (I)+(D.2.5), B-165: (I)+(D.2.6), B-166: (I)+(D.2.7), B-167: (I)+(E.1.1), B-168: (I)+(E.1.2), B-169: (I)+(E.1.3), B-170: (I)+(E.2.1), B-171: (I)+(E.2.2), B-172: (I)+(E.2.3), B-173: (I)+(E.2.4), B-174: (I)+(E.2.5), B-175: (I)+(E.2.6), B-176: (I)+(E.2.7), B-177: (I)+(E.2.8), B-178: (I)+(F.1.1), B-179: (I)+(F.1.2), B-180: (I)+(F.1.3), B-181: (I)+(F.1.4), B-182: (I)+(F.1.5), B-183: (I)+(F.1.6), B-184: (I)+(F.2.1), B-185: (I)+(G.1.1), B-186: (I)+(G.1.2), B-187: (I)+(G.1.3), B-188: (I)+(G.1.4), B-189: (I)+(G.2.1), B-190: (I)+(G.2.2), B-191: (I)+(G.2.3), B-192: (I)+(G.2.4), B-193: (I)+(G.2.5), B-194: (I)+(G.2.6), B-195: (I)+(G.2.7), B-196: (I)+(G.3.1), B-197: (I)+(G.3.2), B-198: (I)+(G.3.3), B-199: (I)+(G.3.4), B-200: (I)+(G.3.5), B-201: (I)+(G.3.6), B-202: (I)+(G.3.7), B-203: (I)+(G.3.8), B-204: (I)+(G.4.1), B-205: (I)+(G.5.1), B-206: (I)+(G.5.2), B-207: (I)+(G.5.3), B-208: (I)+(H.1.1), B-209: (I)+(H.1.2), B-210: (I)+(H.1.3), B-211: (I)+(H.1.4), B-212: (I)+(H.1.5), B-213: (I)+(H.1.6), B-214: (I)+(H.2.1), B-215: (I)+(H.2.2), B-216: (I)+(H.2.3), B-217: (I)+(H.2.4), B-218: (I)+(H.2.5), B-219: (I)+(H.2.6), B-220: (I)+(H.2.7), B-221: (I)+(H.2.8), B-222: (I)+(H.2.9), B-223: (I)+(H.3.1), B-224: (I)+(H.3.2), B-225: (I)+(H.3.3), B-226: (I)+(H.3.4), B-227: (I)+(H.3.5), B-228: (I)+(H.3.6), B-229: (I)+(H.3.7), B-230: (I)+(H.3.8), B-231: (I)+(H.3.9), B-232: (I)+(H.3.10), B-233: (I)+(H.3.11), B-234: (I)+(H.4.1), B-235: (I)+(H.4.2), B-236: (I)+(H.4.3), B-237: (I)+(H.4.4), B-238: (I)+(H.4.5), B-239: (I)+(H.4.6), B-240: (I)+(H.4.7), B-241: (I)+(H.4.8), B-242: (I)+(H.4.9), B-243: (I)+(H.4.10), B-244: (I)+(1.1.1), B-245: (I)+(1.1.2), B-246: (I)+(1.2.1), B-247: (I)+(1.2.2), B-248: (I)+(1.2.3), B-249: (I)+(1.2.4), B-250: (I)+(1.2.5), B-251: (I)+(J.1.1), B-252: (I)+(J.1.2), B-253: (I)+(J.1.3), B-254: (I)+(J.1.4), B-255: (I)+(J.1.5), B-256: (I)+(J.1.6), B-257: (I)+(J.1.7), B-258: (I)+(J.1.8), B-259: (I)+(J.1.9), B-260: (I)+(J.1.10), B-261: (I)+(J.1.11), B-262: (I)+(J.1.12), B-263: (I)+(K.1.1), B-264: (I)+(K.1.2), B-265: (I)+(K.1.3), B-266: (I)+(K.1.4), B-267: (I)+(K.1.5), B-268: (I)+(K.1.6), B-269: (I)+(K.1.7), B-270: (I)+(K.1.8), B-271: (I)+(K.1.9), B-272: (I)+(K.1.10), B-273: (I)+(K.1.11), B-274: (I)+(K.1.12), B-275: (I)+(K.1.13), B-276: (I)+(K.1.14), B-277: (I)+(K.1.15), B-278: (I)+(K.1.16), B-279: (I)+(K.1.17), B-280: (I)+(K.1.18), B-281: (I)+(K.1.19), B-282: (I)+(K.1.20), B-283: (I)+(K.1.21), B-284: (I)+(K.1.22), B-285: (I)+(K.1.23), B-286: (I)+(K.1.24), B-287: (I)+(K.1.25), B-288: (I)+(K.1.26), B-289: (I)+(K.1.27), B-290: (I)+(K.1.28), B-291: (I)+(K.1.29), B-292: (I)+(K.1.30), B-293: (I)+(K.1.31), B-294: (I)+(K.1.32), B-295: (I)+(K.1.33), B-296: (I)+(K.1.34), B-297: (I)+(K.1.35), B-298: (I)+(K.1.36), B-299: (I)+(K.1.37), B-300: (I)+(K.1.38), B-301: (I)+(K.1.39), B-302: (I)+(K.1.40), B-303: (I)+(K.1.41), B-304: (I)+(K.1.42), B-305: (I)+(K.1.43), B-306: (I)+(K.1.44), B-307: (I)+(K.1.45), B-308: (I)+(K.1.47), B-309: (I)+(M.1.1), B-310: (I)+(M.1.2), B-311: (I)+(M.1.3), B-312: (I)+(M.1.4), B-313: (I)+(M.1.5), B-314: (I)+(M.1.6), B-315: (I)+(M.1.7), B-316: (I)+(M.1.8), B-317: (I)+(M.1.9), B-318: (I)+(M.1.10), B-319: (I)+(M.1.11), B-320: (I)+(M.1.12), B-321: (I)+(M.1.13), B-322: (I)+(M.1.14), B-323: (I)+(M.1.15), B-324: (I)+(M.1.16), B-325: (I)+(M.1.17), B-326: (I)+(M.1.18), B-327: (I)+(M.1.19), B-328: (I)+(M.1.20), B-329: (I)+(M.1.21), B-330: (I)+(M.1.22), B-331: (I)+(M.1.23), B-332: (I)+(M.1.24), B-333: (I)+(M.1.25), B-334: (I)+(M.1.26), B-335: (I)+(M.1.27), B-336: (I)+(M.1.28), B-337: (I)+(M.1.29), B-338: (I)+(M.1.30), B-339: (I)+(M.1.31), B-340: (I)+(M.1.32), B-341: (I)+(M.1.33), B-342: (I)+(M.1.34), B-343: (I)+(M.1.35), B-344: (I)+(M.1.36), B-345: (I)+(M.1.37), B-346: (I)+(M.1.38), B-347: (I)+(M.1.39), B-348: (I)+(M.1.40), B-349: (I)+(M.1.41), B-350: (I)+(M.1.42), B-351: (I)+(M.1.43), B-352: (I)+(M.1.44), B-353: (I)+(M.1.45), B-354: (I)+(M.1.46), B-355: (I)+(M.1.47), B-356: (I)+(M.1.48), B-357: (I)+(M.1.49), B-358: (I)+(M.1.50), B-359: (I)+(N.1.1), B-360: (I)+(N.1.2), B-361: (I)+(N.1.3), B-362: (I)+(N.1.4), B-363: (I)+(N.1.5), B-364: (I)+(N.2.1), B-365: (I)+(N.2.2), B-366: (I)+(N.2.3), B-367: (I)+(N.3.1), B-368: (I)+(N.3.2), B-369: (I)+(N.3.3), B-370: (I)+(N.3.4), B-371: (I)+(N.4.1), B-372: (I)+(N.5.1), B-373: (I)+(N.6.1), B-374: (I)+(N.6.2), B-375: (I)+(N.6.3), B-376: (I)+(N.6.4), B-377: (I)+(N.6.5), B-378: (I)+(N.7.1), B-379: (I)+(N.7.2), B-380: (I)+(N.7.3), B-381: (I)+(N.8.1), B-382: (I)+(N.9.1), B-383: (I)+(N.10.1), B-384: (I)+(N.10.2), B-385: (I)+(N.10.3), B-386: (I)+(N.10.4), B-387: (I)+(N.10.5), B-388: (I)+(N.11.1), B-389: (I)+(N.12.1), B-390: (I)+(N.12.2), B-391: (I)+(N.12.3), B-392: (I)+(N.12.4), B-393: (I)+(N.13.1), B-394: (I)+(N.13.2), B-395: (I)+(N.13.3), B-396: (I)+(N.13.4), B-397: (I)+(N.13.5), B-398: (I)+(N.13.6), B-399: (I)+(N.13.7), B-400: (I)+(N.13.8), B-401: (I)+(N.13.9), B-402: (I)+(N.14.1), B-403: (I)+(N.14.2), B-404: (I)+(N.14.3), B-405: (I)+(N.15.1), B-406: (I)+(N.16.1), B-407: (I)+(N.16.2), B-408: (I)+(N.17.1), B-409: (I)+(N.17.2), B-410: (I)+(N.17.3), B-411: (I)+(N.17.4), B-412: (I)+(N.17.5), B-413: (I)+(N.17.6), B-414: (I)+(N.17.7), B-415: (I)+(N.17.8), B-416: (I)+(N.17.9), B-417: (I)+(N.17.10), B-418: (I)+(N.17.11), B-419: (I)+(N.17.12), B-420: (I)+(0.1.1), B-421: (I)+(0.1.2), B-422: (I)+(0.1.3), B-423: (I)+(0.1.4), B-424: (I)+(0.1.5), B-425: (I)+(0.1.6), B-426: (I)+(0.1.7), B-427: (I)+(0.1.8), B-428: (I)+(0.1.9), B-429: (I)+(0.1.10), B-430: (I)+(0.1.11), B-431: (I)+(0.1.12), B-432: (I)+(0.1.13), B-433: (I)+(0.1.14), B-434: (I)+(0.1.15), B-435: (I)+(0.1.16), B-436: (I)+(0.1.17), B-437: (I)+(0.1.18), B-438: (I)+(0.1.19), B-439: (I)+(0.1.20), B-440: (I)+(0.1.21), B-441: (I)+(0.1.22), B-442: (I)+(0.1.23), B-443: (I)+(0.1.24), B-444: (I)+(0.1.25), B-445: (I)+(0.1.26), B-446: (I)+(0.1.27), B-447: (I)+(0.1.28), B-448: (I)+(0.1.29), B-449: (I)+(0.1.30), B-450: (I)+(0.1.31), B-451: (I)+(0.1.32), B-452: (I)+(0.1.33), B-453: (I)+(O.1.34), B-454: (I)+(O.1.35), B-455: (I)+(O.1.36), B-456: (I)+(O.1.37), B-457: (I)+(O.1.38), B-458: (I)+(O.2.1), B-459: (I)+(O.2.2), B-460: (I)+(O.2.3), B-461: (I)+(O.2.4), B-462: (I)+(O.2.5), B-463: (I)+(O.2.6), B-464: (I)+(O.2.7), B-465: (I)+(O.2.8), B-466: (I)+(O.2.9), B-467: (I)+(O.2.10), B-468: (I)+(O.2.11), B-469: (I)+(O.2.12), B-470: (I)+(O.2.13), B-471: (I)+(O.2.14), B-472: (I)+(O.2.15), B-473: (I)+(O.2.16), B-474: (I)+(O.3.1), B-475: (I)+(O.3.2), B-476: (I)+(O.3.3), B-477: (I)+(O.3.4), B-478: (I)+(O.3.5), B-479: (I)+(O.3.6), B-480: (I)+(O.3.7), B-481: (I)+(O3.8), B-482: (I)+(O.3.9), B-483: (I)+(O.3.10), B-484: (I)+(O.3.11), B-485: (I)+(O.3.12), B-486: (I)+(O.3.13), B-487: (I)+(O.3.14), B-488: (I)+(O.3.15), B-489: (I)+(O.3.16), B-490: (I)+(O.3.17), B-491: (I)+(O.3.18), B-492: (I)+(O.3.19), B-493: (I)+(O.3.20), B-494: (I)+(O.3.21), B-495: (I)+(O.3.22), B-496: (I)+(O.3.23), B-497: (I)+(O.3.24), B-498: (I)+(O.3.25), B-499: (I)+(O.3.26), B-500: (I)+(O.3.27), B-501: (I)+(O.4.1), B-502: (I)+(O.4.2), B-503: (I)+(O.4.3), B-504: (I)+(O.4.4), B-505: (I)+(O.4.5), B-506: (I)+(O.4.6), B-507: (I)+(O.4.7), B-508: (I)+(O.4.8), B-509: (I)+(O.4.9), B-510: (I)+(O.4.10), B-511: (I)+(O.4.11), B-512: (I)+(O.4.12), B-513: (I)+(O.4.13), B-514: (I)+(O.4.14), B-515: (I)+(O.4.15), B-516: (I)+(O.4.16), B-517: (I)+(O.4.17), B-518: (I)+(O.4.18), B-519: (I)+(O.4.19), B-520: (I)+(O.4.20), B-521: (I)+(O.4.21), B-522: (I)+(O.4.22), B-523: (I)+(O.4.23), B-524: (I)+(O.4.24), B-525: (I)+(O.5.1), B-526: (I)+(O.5.2), B-527: (I)+(O.5.3), B-528: (I)+(O.5.4), B-529: (I)+(O.5.5), B-530: (I)+(O.5.6), B-531: (I)+(O.5.7), B-532: (I)+(O.5.8), B-533: (I)+(O.5.9), B-534: (I)+(O.6.1), B-535: (I)+(O.6.2), B-536: (I)+(O.6.3), B-537: (I)+(O.6.4), B-538: (I)+(O.6.5), B-539: (I)+(O.6.6), B-540: (I)+(O.6.7), B-541: (I)+(O.7.1), B-542: (I)+(O.7.2), B-543: (I)+(O.7.3), B-544: (I)+(O.7.4), B-545: (I)+(O.7.5), B-546: (I)+(O.7.6), B-547: (I)+(O.8.1), B-548: (I)+(O.8.2), B-549: (I)+(O.8.3), B-550: (I)+(O.8.4), B-551: (I)+(O.8.5), B-552: (I)+(O.9.1), B-553: (I)+(O.9.2), B-554: (I)+(O.9.3), B-555: (I)+(O.10.1), B-556: (I)+(O.11.1), B-557: (I)+(O.11.2), B-558: (I)+(O.11.3), B-559: (I)+(O.11.4), B-560: (I)+(O.12.1), B-561: (I)+(O.13.1), B-562: (I)+(O.14.1), B-563: (I)+(O.14.2), B-564: (I)+(O.15.1), B-565: (I)+(O.15.2), B-566: (I)+(O.15.3), B-567: (I)+(O.15.4), B-568: (I)+(O.15.5), B-569: (I)+(O.15.6), B-570: (I)+(O.15.7), B-571: (I)+(O.15.8), B-572: (I)+(O.15.9), B-573: (I)+(O.15.10), B-574: (I)+(O.15.11), B-575: (I)+(O.16.1), B-576: (I)+(O.16.2), B-577: (I)+(O.16.3), B-578: (I)+(O.16.4), B-579: (I)+(O.16.5), B-580: (I)+(O.16.6), B-581: (I)+(O.17.1), B-582: (I)+(O.18.1), B-583: (I)+(O.18.2), B-584: (I)+(O.18.3), B-585: (I)+(O.18.4), B-586: (I)+(O.18.5), B-587: (I)+(O.19.1), B-588: (I)+(O.20.1), B-589: (I)+(O.20.2), B-590: (I)+(O.20.3), B-591: (I)+(O.21.1), B-592: (I)+(O.21.2), B-593: (I)+(O.21.3), B-594: (I)+(O.21.4), B-595: (I)+(O.21.5), B-596: (I)+(O.21.6), B-597: (I)+(O.21.7), B-598: (I)+(O.22.1), B-599: (I)+(O.22.2), B-600: (I)+(O.22.3), B-601: (I)+(O.22.4), B-602: (I)+(O.23.1), B-603: (I)+(O.23.2), B-604: (I)+(O.23.3), B-605: (I)+(O.24.1), B-606: (I)+(O.24.2), B-607: (I)+(O.24.3), B-608: (I)+(O.24.4), B-609: (I)+(O.24.5), B-610: (I)+(O.25.1), B-611: (I)+(O.25.2), B-612: (I)+(O.26.1), B-613: (I)+(O.26.2), B-614: (I)+(O.26.3), B-615: (I)+(O.26.4), B-616: (I)+(O.26.5), B-617: (I)+(O.26.6), B-618: (I)+(O.26.7), B-619: (I)+(O.26.8), B-620: (I)+(O.26.9), B-621: (I)+(O.26.10), B-622: (I)+(O.26.11), B-623: (I)+(O.26.12), B-624: (I)+(O.26.13), B-625: (I)+(O.26.14), B-626: (I)+(O.26.15), B-627: (I)+(O.26.16), B-628: (I)+(O.26.17), B-629: (I)+(O.26.18), B-630: (I)+(O.27.1), B-631: (I)+(O.27.2), B-632: (I)+(O.27.3), B-633: (I)+(O.27.4), B-634: (I)+(O.27.5), B-635: (I)+(O.27.6), B-636: (I)+(O.27.7), B-637: (I)+(O.27.8), B-638: (I)+(O.27.9), B-639: (I)+(O.27.10), B-640: (I)+(O.27.11), B-641: (I)+(O.27.12), B-642: (I)+(O.27.13), B-643: (I)+(O.27.14), B-644: (I)+(O.27.15), B-645: (I)+(O.27.16), B-646: (I)+(O.27.17), B-647: (I)+(O.27.18), B-648: (I)+(O.27.19), B-649: (I)+(O.27.20), B-650: (I)+(O.27.21), B-651: (I)+(O.27.22), B-652: (I)+(O.27.23), B-653: (I)+(O.27.24), B-654: (I)+(O.27.25), B-655: (I)+(O.27.26), B-656: (I)+(O.27.27), B-657: (I)+(O.27.28), B-658: (I)+(O.27.29), B-659: (I)+(O.27.30), B-660: (I)+(O.27.31), B-661: (I)+(O.27.32), B-662: (I)+(O.27.33), B-663: (I)+(O.27.34), B-664: (I)+(O.27.35), B-665: (I)+(O.27.36), B-666: (I)+(O.27.37), B-667: (I)+(O.27.38), B-668: (I)+(O.27.39), B-669: (I)+(O.27.40), B-670: (I)+(O.27.41), B-671: (I)+(O.27.42), B-672: (I)+(O.27.43), B-673: (I)+(O.27.44), B-674: (I)+(O.27.45), B-675: (I)+(O.27.46), B-676: (I)+(O.27.47), B-677: (I)+(O.27.48), B-678: (I)+(O.27.49), B-679: (I)+(O.27.50), B-680: (I)+(O.27.51), B-681: (I)+(O.27.52), B-682: (I)+(O.27.53), B-683: (I)+(O.27.54), B-684: (I)+(O.27.55), B-685: (I)+(O.27.56), B-686: (I)+(O.27.57), B-687: (I)+(O.27.58), B-688: (I)+(O.27.59), B-689: (I)+(O.27.60), B-690: (I)+(O.27.61), B-691: (I)+(O.27.62), B-692: (I)+(O.27.63), B-693: (I)+(O.27.64), B-694: (I)+(O.27.65), B-695: (I)+(O.27.66), B-696: (I)+(O.27.67), B-697: (I)+(O.27.68), B-698: (I)+(O.27.69), B-699: (I)+(O.27.70), B-700: (I)+(O.27.71), B-701: (I)+(O.27.72), B-702: (I)+(O.27.73), B-703: (I)+(O.27.74), B-704: (I)+(O.27.75), B-705: (I)+(O.27.76), B-706: (I)+(O.27.77), B-707: (I)+(O.27.78), B-708: (I)+(O.27.79), B-709: (I)+(O.27.80), B-710: (I)+(O.27.81), B-711: (I)+(O.27.82), B-712: (I)+(O.27.83), B-713: (I)+(O.27.84), B-714: (I)+(O.27.85), B-715: (I)+(O.27.86), B-716: (I)+(O.27.87), B-717: (I)+(O.27.88), B-718: (I)+(O.27.89), B-719: (I)+(O.27.90), B-720: (I)+(O.27.91), B-721: (I)+(O.27.92), B-722: (I)+(O.27.93), B-723: (I)+(O.27.94), B-724: (I)+(O.27.95), B-725: (I)+(O.27.96), B-726: (I)+(O.27.97), B-727: (I)+(O.27.98), B-728: (I)+(O.27.99), B-729: (I)+(O.27.100).

The mixtures of active substances can be prepared as compositions comprising besides the active ingredients at least one inert ingredient (auxiliary) by usual means, e. g. by the means given for the compositions of compounds I. Concerning usual ingredients of such compositions reference is made to the explanations given for the compositions containing compounds I. The mixtures of active substances according to the present invention are suitable as fungicides, as are the compounds of formula I. They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes). In addition, it is refered to the explanations regarding the fungicidal activity of the compounds and the compositions containing compounds I, respectively.

### I. Synthesis examples

The compounds of formula I can be prepared according to the methods outlined below.

### I.1) Preparation of 4-(ethylsulfanylmethyl)benzonitrile

A solution of 4-(bromomethyl)benzonitrile (1.24 g, 6.3 mmol), sodium thioethylate (0.53 g, 1 eq.) and 10 mL *N,N-*dimethylformamide was heated for 2 hours at 60 °C. After cooling to room temperature, the solvent was removed under reduced pressure. The title compound was used directly without further purification.

### I.2) Preparation of 3-[4-(ethylsulfanylmethyl)phenyl]-5-(trifluoromethyl)-1,2,4-oxadiazole

### (Ex-25)

A solution of 4-(ethylsulfanylmethyl)benzonitrile as obtained from above (1.11 g, 1 eq.) and trimethylamine (1.58 g, 2.5 eq.) in tetrahydrofuran (100 mL), hydroxylamine hydrochloride (0,65 g, 1.5 eq.) was added. The mixture was stirred at room temperature until complete conversion of starting material was detected via high pressure liquid chromatography. Trifluoroacetic anhydride (3.16 g, 2.4 eq.) was added slowly and the mixture was stirred for 30 minutes. After cooling to room temperature the mixture was poured into the water, the aqueous solution was extracted with ethylacetate and was washed with a sodium chloride solution (10 %). The combined organic layer was dried with magnesium sulfate and freed from solvent. The residue was purified by column chromatography (cyclohexane : ethyl acetate) to afford the desired product as a colorless oil (154 mg, 8 % yield). ¹H NMR (500 MHz, CDCl₃): δ [ppm] = 8.1 (arom. *CH,* 2H), 7.5 (arom. *CH,* 2H), 3.8 (*CH₂S,* 2H), 2.4 (*CH₂, 2H*), 1.3 (*CH₃,* 3H).

### I.3) Preparation of 3-[4-(ethylsulfonylmethyl)phenyl]-5-(trifluoromethyl)-1,2,4-oxadiazole

### (Ex-17)

A solution of 3-[4-(ethylsulfanylmethyl)phenyl]-5-(trifluoromethyl)-1,2,4-oxadiazole (119 mg, 1 eq.) in dichloromethane (1.5 mL) was cooled to 0 °C and *m*-chloroperbenzoic acid (213 mg, 3 eq.) was added. The mixture was stirred over night at room temperature and an aqueous solution of sodium thiosulfate was added. The aqueous solution was extracted with ethylacetate and was washed with a sodium chloride solution (10 %). The combined organic layer was dried with magnesium sulfate and freed from solvent. The residue was purified by column chromatography (cyclohexane : ethyl acetate) to afford the desired product as a colorless solid material. Melting point: 164 °C; ¹H NMR (500 MHz, CDCl₃): δ [ppm] = 8.2 (arom. CH, 2H), 7.6 (arom. CH, 2H), 4.3 (CH₂S, 2H), 2.9 (CH₂, 2H), 1.4 (CH₃, 3H).

The compounds listed in Table I were prepared in an analogous manner.

**Table I: Compounds Ex-1 to Ex-30 of the formula I.X, wherein the meaning of -[CR²R³]ₙ-, L and R¹ are as defined in each line.**

| | | | | | |
|---|---|---|---|---|---|
| 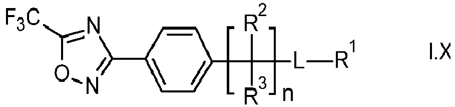 | | | | | |

| **Ex. no** | 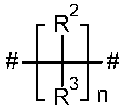 | L | **R**¹ | **HPLC Rₜ (min)*; NMR: 500 mHz, CDCl₃, 298K, [ppm]** | **Melting point (°C)** |
|---|---|---|---|---|---|
| Ex-1 | n = 0 | -S(=O)- | methyl | 0.987 | 111 |
| Ex-2 | n = 0 | -S(=O)₂- | methyl | - | 150 |
| Ex-3 | n = 0 | -S(=O)- | benzyl | 1.202 | 181 |
| Ex-4 | n = 0 | -S(=O)₂- | benzyl | - | 207 |
| Ex-5 | n = 0 | -S(=O)₂- | phenyl | 1.270 | 142 |
| Ex-6 | n = 0 | -S(=O)₂- | cyclopropyl | - | 108 |
| Ex-7 | n = 0 | -S(=O)₂- | 1-methylpropyl | 1.243 | oil |
| Ex-8 | n = 0 | -S(=O)₂- | 4-F-phenyl | - | 156 |
| Ex-9 | n = 0 | -S(=O)₂- | 2-F-phenyl | - | 150 |
| Ex-10 | n = 0 | -S(=O)₂- | 2,4-difluorphenyl | - | 128 |
| Ex-11 | n = 1; R² and R³ = H | -S(=O)₂- | 2-F-phenyl | - | 154 |
| Ex-12 | n = 1; R² and R³ = H | -S(=O)₂- | 4-F-phenyl | - | 203 |
| Ex-13 | n = 1; R² and R³ = H | -S(=O)₂- | phenyl | - | 225 |
| Ex-14 | n = 1; R² and R³ = H | -S(=O)₂- | iso-propyl | - | 164 |
| Ex-15 | n = 1; R² and R³ = H | -S- | 2,4-difluorphenyl | - | 48 |
| Ex-16 | n = 1; R² and R³ = H | -S(=O)₂- | 2,4-difluorphenyl | - | 163 |
| Ex-17 | n = 1; R² and R³ = H | -S(=O)₂- | ethyl | - | 164 |
| Ex-18 | n = 0 | 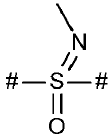 | methyl | 0.882 | oil |
| Ex-19 | n = 0 | 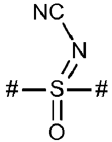 | methyl | 1.012 | 163 |
| Ex-20 | n = 0 | 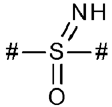 | methyl | 0.891 | 120 |
| Ex-21 | n = 1; R² and R³ = H | -S- | phenyl | - | 92 |
| Ex-22 | n = 1; R² and R³ = H | -S- | 4-F-phenyl | - | 64 |
| Ex-23 | n = 1; R² and R³ = H | -S- | 2-F-phenyl | - | 62 |
| Ex-24 | n = 1; R² and R³ = H | -S- | iso-propyl | 1.455 | 34 |
| Ex-25 | n = 1; R² and R³ = H | -S- | ethyl | [8.1, 7.5, 3.8, 2.4, 1.3] | oil |
| Ex-26 | n = 1; R² and R³ = H | -S- | methyl | [7.6, 7.4, 3.7, 1.9] | oil |
| Ex-27 | n = 0 | -S- | methyl | 1.328 | 71 |
| Ex-28 | n = 0 | -S- | benzyl | 1.471 | 91 |
| Ex-29 | n = 0 | -S- | 1-methylpropyl | 1.525 | oil |
| Ex-30 | n = 1; R² and R³ = H | -S(=O)₂- | methyl | - | 141 |

| | | | | | |
|---|---|---|---|---|---|
| * HPLC: High Performance Liquid Chromatography; HPLC-column Kinetex XB C18 1,7µ (50 x 2,1 mm); eluent: acetonitrile / water + 0.1 % trifluoroacetic acid (gradient from 5:95 to 100 : 0 in 1.5 min at 60°C, flow gradient from 0.8 to 1.0 ml/min in 1.5 min). MS: Quadrupol Electrospray lonisation, 80 V (positive mode). Rₜ: retention time in minutes. | | | | | |

## Claims

1. Compounds of the formula I, or the N-oxides, or the agriculturally acceptable salts thereof 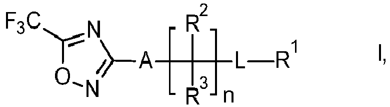 wherein:
A is phenyl; and wherein the phenyl ring is unsubstituted or substituted with 1, 2, 3 or 4 identical or different groups R^{A}; wherein
R^{A} is halogen, cyano, C₁-C₆-alkyl, C₁-C₆-haloalkyl, C₁-C₆-alkoxy or C₁-C₆-haloalkoxy;
L is -S(=O)ₚ- or -S(=N-R^{L})(=O)-;
p is 0, 1 or 2;
R^{L} is hydrogen, cyano or C₁-C₃-alkyl;
R¹ is C₁-C₆-alkyl, C₁-C₆-alkoxy, C₂-C₆-alkenyl, C₂-C₆-alkynyl, C₃-C₈-cycloalkyl, C₃-C₈-cycloalkoxy, C₃-C₈-cycloalkenyl, phenyl-C₁-C₄-alkyl, heteroaryl-C₁-C₄-alkyl, phenyl, naphthyl or a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms; and wherein 1 or 2 carbon ring member atoms of the heterocycle may be replaced by 1 or 2 groups independently selected from -C(=O)- and -C(=S)-; and wherein the heteroaryl group in heteroaryl-C₁-C₄-alkyl is a 5- or 6-membered aromatic heterocycle, wherein the ring member atoms of the heterocyclic ring include besides carbon atoms 1, 2, 3 or 4 heteroatoms selected from N, O and S as ring member atoms; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl, C₁-C₆-haloalkyl, C₁-C₆-alkoxy, C₁-C₆-haloalkoxy and C₃-C₈-cycloalkyl;
R², R³ each single radical individually and independently of each other, are selected from the group consisting of hydrogen, halogen, cyano, C₁-C₄-alkyl, C₁-C₄-alkenyl, C₁-C₄-alkynyl, C₁-C₄-haloalkyl, C₁-C₄-alkoxy and C₁-C₄-haloalkoxy;
or R² and R³ together with the carbon atom to which they are bound form a saturated 3- to 7-membered carbocycle or a saturated 3- to 6-membered heterocycle; wherein the saturated heterocycle includes beside carbon atoms 1, 2 or 3 heteroatoms independently selected from the group consisiting of N, O and S as ring member atoms; and wherein said N ring member atom is substituted with the group R^{N}; and wherein
R^{N} is hydrogen, C₁-C₆-alkyl or halogen;
and wherein said S ring member atom is unsubstituted or substituted with 1 or 2 oxo radicals; and wherein one or two CH₂ groups of the saturated carbocycle or of the saturated heterocycle may be replaced by one or two groups independently selected from -C(=O)- and -C(=S)-; and wherein the carbon ring member atoms of the saturated carbocycle or the saturated heterocycle are unsubstituted or substituted with a total number of 1, 2, 3, 4 or up to the maximum possible number of identical or different radicals selected from the group consisting of halogen, cyano, C₁-C₆-alkyl, C₁-C₆-haloalkyl, C₁-C₆-alkoxy, C₁-C₆-haloalkoxy and C₃-C₈-cycloalkyl; and
n is 0, 1 or 2.

2. Compounds according to claim 1, wherein the -CR²R³-L-R¹ group is attached to the phenyl ring A in para-position with regard to the trifluoromethyloxadiazole group.

3. Compounds according to claim 1 or 2, wherein L is -S(=O)₂-.

4. Compounds according to any one of claims 1 to 3, wherein n is 0 or 1.

5. Compounds according to any one of claims 1 to 4, wherein R¹ is C₁-C₆-alkyl, C₃-C₈-cycloalkyl, phenyl-C₁-C₄-alkyl or phenyl; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl, C₁-C₆-haloalkyl, C₁-C₆-alkoxy, C₁-C₆-haloalkoxy and C₃-C₈-cycloalkyl.

6. Compounds according to any one of claims 1 to 4, wherein R¹ is C₁-C₆-alkyl, C₁-C₆-haloalkyl, C₃-C₆-cycloalkyl or phenyl; wherein the cyclic groups are unsubstituted or substituted with 1, 2, 3 or 4 or up to the maximum possible number of identical or different radicals selected from the group consisiting of chlorine, fluorine, methyl, methoxy, CF₃ or OCF₃.

7. Compounds according to any one of claims 1 to 6, wherein A is unsubstituted or substituted with 1 or 2 identical or different groups R^{A}; and wherein R^{A} is fluorine, chlorine or C₁-C₆-alkyl.

8. Compounds according to any one of claims 1 to 7, wherein R² and R³, each single radical individually and independently of each other, are selected from the group consisting of hydrogen, fluorine and C₁-C₄-alkyl.

9. A mixture comprising at least one compound of the formula I according to any one of claims 1 to 8 and at least one further pesticidally active substance selected from the group consisting of herbicides, safeners, fungicides, insecticides, and plant growth regulators.

10. An agrochemical composition, which comprises an auxiliary and at least one compound of the formula I, or an N-oxide, or an agriculturally acceptable salt thereof, as defined in any one of claims 1 to 8.

11. An agrochemical composition according to claim 10, wherein the auxiliary is selected from the group of ionic or non-ionic surfactants.

12. An agrochemical composition according to any one of claims 10 or 11 comprising at least one further pesticidally active substance selected from the group consisting of herbicides, safeners, fungicides, insecticides, and plant growth regulators.

13. An agrochemical composition according to any one of claims 10 to 12, further comprising seed, wherein the amount of the compound of the formula I, or an N-oxide, or an agriculturally acceptable salt thereof, is from 0.1 g to 10 kg per 100 kg of seed.

14. The use of compounds according to any one of claims 1 to 8 for combating phytopathogenic harmful fungi.

15. A method for combating phytopathogenic harmful fungi, which process comprises treating the fungi, the plants, the soil or seeds to be protected against fungal attack, with an effective amount of at least one compound of formula I, or an N-oxide, or an agriculturally acceptable salt thereof, as defined in any one of claims 1 to 8.
